# EUROPÄISCHE PATENTSCHRIFT

(11) **EP 1 412 509 B1**
(45) Veröffentlichungstag und Bekanntmachung des Hinweises auf die Patenterteilung: **10.01.2007**
(21) Anmeldenummer: 02767074.4
(22) Anmeldetag: 29.07.2002
(51) Int. Cl.: C12N 15/85

(54) **RNA-AMPLIFIKATIONSSYSTEM MIT KOMPONENTEN AUS DER PFLANZE IN TIERISCHEN ZELLEN**
RNA AMPLIFICATION SYSTEM USING PLANT COMPONENTS IN ANIMAL CELLS
SYSTEME D'AMPLIFICATION D'ARN AVEC DES COMPOSANTES VEGETALES DANS DES CELLULES ANIMALES

(30) Priorität: 30.07.2001 DE 10137444
(43) Veröffentlichungstag der Anmeldung: 28.04.2004
(73) Patentinhaber: ProBioGen AG, 13086 Berlin (DE)
(72) Erfinder: SANDIG, Volker, 13189 Berlin (DE); JORDAN, Ingo, 10409 Berlin (DE)
(74) Vertreter: Helbing, Jörg
(86) Internationale Anmeldenummer: PCT/DE2002/002863
(87) Internationale Veröffentlichungsnummer: WO 2003/014366

(56) Entgegenhaltungen:
- WO-A-99/49085
- US-A- 5 556 769
- US-B1- 6 218 142
- NAGY PETER D ET AL: "RNA elements required for RNA recombination function as replication enhancers in vitro and in vivo in a plus-strand RNA virus." EMBO (EUROPEAN MOLECULAR BIOLOGY ORGANIZATION) JOURNAL, Bd. 18, Nr. 20, 15. Oktober 1999 (1999-10-15), Seiten 5653-5665, XP002243959 ISSN: 0261-4189

### SEQUENZPROTOKOLL

<110> ProBioGen AG
<120>
   RNA-AMPLIFIKATIONSSYSTEM MIT KOMPONENTEN AUS DER PFLANZE IN TIERISCHEN ZELLEN
SEQUENZ #1 (DNA)
   (RdRP; interne TAG-Deletion und interenes ATG hervorgehoben)
SEQUENZ #1 (TRANSLATIERT)
SEQUENZ #2
   (SatC, sense-Orientierung; interne ATGs und BstE II Schnittstelle hervorgehoben).
SEQUENZ #3
   (SatC, anti sense-Orientierung; potentielles Polyadenylierungssignal und BstE II hervorgehoben).
SEQUENZ#4 (pIJO-20)
SEQUENZ #5 (pIJO-79)

### SEQUENZPROTOKOLL

<110> ProBioGen AG
   Sandig, Volker
   Jordan, Ingo
<120> RNA-Amplifikationssystem mit Komponenten aus der
   Pflanze in tierischen Zellen
<130> PCT/DE 02/02863
<140> PCT/DE 02/02863
   <141> 2002-07-29
<150> DE 101 37 444.5-41
   <151> 2001-07-30
<160> 6
<170> Patent In Ver. 2.1
<210> 1
   <211> 2325
   <212> DNA
   <213> Turnip crinkle virus
<400> 1
   atgcctcttc tacacacact caacacagcg ctcgcagtgg gactcctagg agccaggtac 60
   taccctgagg ttcaaacctt cttggggctg cctgactacg tgggtcacat gaagaatgta 120
   gtacggtctg ttttccaggg atctgggcta gtagtagtgt cctccgacac agtcggtgtc 180
   agggggacgt atagtaatag aggtcagata ggtagtagtc tcgggtgtat actagccgtt 240
   ccggatagcg gggcggatat agaaatagac ctagataggt tggtaggaac ggaagaggaa 300
   gccacatcct gtttggtgga ggcggtaggt agtaccgcag atgtccccag gaggagagtt 360
   cgtcaaaagg ggcggtttgc tatgcatgcc gtcaacgcag caaagctgca cttttgtggc 420
   gtcccaaaac ccactgaagc gaatcgacta gcggtctcaa aatggcttgt ccaatactgc 480
   aaagagagac atgtcgtaga cagccacatc agaacgatag tcaatacggc tcttcctaga 540
   gtgttcacgc ctgacgcgga agacattcag gtcgtgctgg atttgcacag tgtaagagca 600
   cacgaccacc gcaacgccct agccgaagca ggcaaagtgc ggaagtggtg ggtcaatctc 660
   gcgatgcatc ccatgactgg gaggtcgtgg tccagggctt ggaggcgatt atgccgactg 720
   cctgacgacc aggcgatctc ttttgtccgc gggtgcttgc gggagctggt cgggagggag 780
   actcaaatct ccaggggtga aaacccggcc atgcgcgtgt tcccgttagc aaatcccccg 840
   aaggttcgac gcatcttcca tatctgtgga atgggcaatg gtttagactt tggagtccac 900
   aacaactcac tcaacaattt gagaagaggg ttgatggaaa gagtctttta cgttgaagat 960
   gcgcagaagc aattgaaacc agccccccaa ccgatcccag ggattttcgg gaagttgagt 1020
   gggattcgga gacgattggt caggttggcc ggaaatcata cccctgtgcc tcgggagaaa 1080
   tacccgtcgt tctacaaggg caggagggcc accatatacc aaaaggcttt ggattctcta 1140
   catgacagac cggtatcccg gaaggacgca gaactcaaaa cattcgtgaa ggcagaaaag 1200
   atcaatttca cggctaagaa agacccggct ccacgggtca tccagccgag ggacccacga 1260
   tataacattg aggttgggaa atacttgaaa ccgtacgagc accatttata tcgggcaatt 1320
   gacgctatgt ggggtgggcc cactgtgctg aaaggatacg atgtggggga gcttggaaac 1380
   attatgagta acacctggga taaattccgg aaaacgtgtg cgataggatt tgacatgaag 1440
   agattcgacc agcacgtatc cgtggacgcc ctacgatggg aacacagtgt atacaacgcg 1500
   ggctttaact gtcccgagtt ggcacagctg ctaacttggc agttgaccaa caagggagtt 1560
   gggagagcct ccgatggctt tatcaaatac caagttgatg gttgtcgcat gtccggagat 1620
   gttaacacag ccttgggcaa ctgcctactg gcttgctcta tcaccaagta cttaatgaag 1680
   ggaatcaaat gcaaattaat caacaatgga gacgattgtg tgctgttctt cgaagctgat 1740
   gaagtcgaca gggtgcgcga aaggctgcat cattggatcg actttgggtt tcaatgcata 1800
   gcggaagaac cacaatacga attggagaaa gttgaatttt gccagatgtc ccctattttc 1860
   gatggtgaag ggtgggtcat ggtcagaaac ccccgtgtga gcctctccaa ggacagctac 1920
   agcaccacac aatgggcgaa tgagaaagat gcagccagat ggttggctgc catcggagag 1980
   tgtggcttgg ctattgcagg tggcgtacca gtgttacaat catattattc ttgcctgaag 2040
   aggaattttg gacccctggc cggggactac aagaagaaga tgcaagatgt ttcctttgat 2100
   agtggattct acaggttatc caagaacggg atgaggggca gcaaagacgt gtcccaagat 2160
   gctaggttca gcttttaccg ggggttcggc tacactccag acgagcagga agcgcttgag 2220
   gagtactacg acaacctcga actgctctgt gagtggaccc ccacgggata taaagaagaa 2280
   cttagtgata gatggatcct gaacgaattc cctacaactc tctaa                 2325
<210> 2
   <211> 778
   <212> PRT
   <213> Turnip crinkle virus
<400> 2
   Met Pro Leu Leu His Thr Leu Asn Thr Ala Leu Ala Val Gly Leu Leu
     1               5                  10                  15
   Gly Ala Arg Tyr Tyr Pro Glu Val Gln Thr Phe Leu Gly Leu Pro Asp
                20                  25                  30
   Tyr Val Gly His Met Lys Asn Val Val Arg Ser Val Phe Gln Gly Ser
            35                  40                  45
   Gly Leu Val Val Val Ser Ser Asp Thr Val Gly Val Arg Gly Thr Tyr
        50                  55                  60
   Ser Asn Arg Gly Gln Ile Gly Ser Ser Leu Gly Cys Ile Leu Ala Val
    65                  70                  75                  80
   Pro Asp Ser Gly Ala Asp Ile Glu Ile Asp Leu Asp Arg Leu Val Gly
                    85                  90                  95
   Thr Glu Glu Glu Ala Thr Ser Cys Leu Val Glu Ala Val Gly Ser Thr
               100                 105                 110
   Ala Asp Val Pro Arg Arg Arg Val Arg Gln Lys Gly Arg Phe Ala Met
   115 120 125
   His Ala Val Asn Ala Ala Lys Leu His Phe Cys Gly Val Pro Lys Pro
   130 135 140
   Thr Glu Ala Asn Arg Leu Ala Val Ser Lys Trp Leu Val Gln Tyr Cys
   145 150 155 160
   Lys Glu Arg His Val Val Asp Ser His Ile Arg Thr Ile Val Asn Thr
   165 170 175
   Ala Leu Pro Arg Val Phe Thr Pro Asp Ala Glu Asp Ile Gln Val Val
   180 185 190
   Leu Asp Leu His Ser Val Arg Ala His Asp His Arg Asn Ala Leu Ala
   195 200 205
   Glu Ala Gly Lys Val Arg Lys Trp Trp Val Asn Leu Ala Met His Pro
   210 215 220
   Met Thr Gly Arg Ser Trp Ser Arg Ala Trp Arg Arg Leu Cys Arg Leu
   225 230 235 240
   Pro Asp Asp Gln Ala Ile Ser Phe Val Arg Gly Cys Leu Arg Glu Leu
   245 250 255
   Val Gly Arg Glu Thr Gln Ile Ser Arg Gly Glu Asn Pro Ala Met Arg
   260 265 270
   Val Phe Pro Leu Ala Asn Pro Pro Lys Val Arg Arg Ile Phe His Ile
   275 280 285
   Cys Gly Met Gly Asn Gly Leu Asp Phe Gly Val His Asn Asn Ser Leu
   290 295 300
   Asn Asn Leu Arg Arg Gly Leu Met Glu Arg Val Phe Tyr Val Glu Asp
   305 310 315 320
   Ala Gln Lys Gln Leu Lys Pro Ala Pro Gln Pro Ile Pro Gly Ile Phe
   325 330 335
   Gly Lys Leu Ser Gly Ile Arg Arg Arg Leu Val Arg Leu Ala Gly Asn
   340 345 350
   His Thr Pro Val Pro Arg Glu Lys Tyr Pro Ser Phe Tyr Lys Gly Arg
   355 360 365
   Arg Ala Thr Ile Tyr Gln Lys Ala Leu Asp Ser Leu His Asp Arg Pro
   370 375 380
   Val Ser Arg Lys Asp Ala Glu Leu Lys Thr Phe Val Lys Ala Glu Lys
   385 390 395 400
   Ile Asn Phe Thr Ala Lys Lys Asp Pro Ala Pro Arg Val Ile Gln Pro
   405 410 415
   Arg Asp Pro Arg Tyr Asn Ile Glu Val Gly Lys Tyr Leu Lys Pro Tyr
   420 425 430
   Glu His His Leu Tyr Arg Ala Ile Asp Ala Met Trp Gly Gly Pro Thr
   435 440 445
   Val Leu Lys Gly Tyr Asp Val Gly Glu Leu Gly Asn Ile Met Ser Asn
   450 455 460
   Thr Trp Asp Lys Phe Arg Lys Thr Cys Ala Ile Gly Phe Asp Met Lys
   465 470 475 480
   Arg Phe Asp Gln His Val Ser Val Asp Ala Leu Arg Trp Glu His Ser
   485 490 495
   Val Tyr Asn Ala Gly Phe Asn Cys Pro Glu Leu Ala Gln Leu Leu Thr
   500 505 510
   Trp Gln Leu Thr Asn Lys Gly Val Gly Arg Ala Ser Asp Gly Phe Ile
   515 520 525
   Lys Tyr Gln Val Asp Gly Cys Arg Met Ser Gly Asp Val Asn Thr Ala
   530 535 540
   Leu Gly Asn Cys Leu Leu Ala Cys Ser Ile Thr Lys Tyr Leu Met Lys
   545 550 555 560
   Gly Ile Lys Cys Lys Leu Ile Asn Asn Gly Asp Asp Cys Val Leu Phe
   565 570 575
   Phe Glu Ala Asp Glu Val Asp Arg Val Arg Glu Arg Leu His His Trp
   580 585 590
   Ile Asp Phe Gly Phe Gln Cys Ile Ala Glu Glu Pro Gln Tyr Glu Leu
   595 600 605
   Glu Lys Val Glu Phe Cys Gln Met Ser Pro Ile Phe Asp Gly Glu Gly
   610 615 620
   Trp Val Met Val Arg Asn Pro Arg Val Ser Leu Ser Lys Asp Ser Tyr
   625                 630                 635                 640
   Ser Thr Thr Gln Trp Ala Asn Glu Lys Asp Ala Ala Arg Trp Leu Ala
                   645                 650                 655
   Ala Ile Gly Glu Cys Gly Leu Ala Ile Ala Gly Gly Val Pro Val Leu
               660                 665                 670
   Gln Ser Tyr Tyr Ser Cys Leu Lys Arg Asn Phe Gly Pro Leu Ala Gly
           675                 680                 685
   Asp Tyr Lys Lys Lys Met Gln Asp Val Ser Phe Asp Ser Gly Phe Tyr
       690                 695                 700
   Arg Leu Ser Lys Asn Gly Met Arg Gly Ser Lys Asp Val Ser Gln Asp
   705                 710                 715                 720
   Ala Arg Phe Ser Phe Tyr Arg Gly Phe Gly Tyr Thr Pro Asp Glu Gln
                   725                 730                 735
   Glu Ala Leu Glu Glu Tyr Tyr Asp Asn Leu Glu Leu Leu Cys Glu Trp
               740                 745                 750
   Asp Pro Thr Gly Tyr Lys Glu Glu Leu Ser Asp Arg Trp Ile Leu Asn
           755                 760                 765
   Glu Phe Pro Thr Thr Leu Gly Lys Tyr Leu
       770                 775
<210> 3
   <211> 402
   <212> DNA
   <213> Turnip crinkle virus
<400> 3
   cccgggataa ctaagggttt catacgatac cacgcaacta atgcagaaca cccattgccc 60
   taaacttgac tgatgaccct tacgtaggcc gggggggttt aaccatggtg ggttgtgaag 120
   gcgggagttc ccatcaagta cgggggcgtg aaacctgaca gtatcccact cgaaagagtc 180
   ttctcctctc tcttatatta agaaaaggaa acaaaacccc caggtcgctt tattttgacc 240
   tgtgttaggg accaaaaacg gtggcagcac tgtctagctg cgggcattag actggaaaac 300
   tagtgctctt tgggtaacca ctaaaatccc gaaagggtgg gctgtgggga ccttccgaac 360
   taaaagatag cctccctcct cgcggggggg ggcctgcccg gg                   402
<210> 4
   <211> 402
   <212> DNA
   <213> Turnip crinkle virus
<400> 4
   cccgggcagg cccccccccg cgaggaggga ggctatcttt tagttcggaa ggtccccaca 60
   gcccaccctt tcgggatttt agtggttacc caaagagcac tagttttcca gtctaatgcc 120
   cgcagctaga cagtgctgcc accgtttttg gtccctaaca caggtcaaaa taaagcgacc 180
   tgggggtttt gtttcctttt cttaatataa gagagaggag aagactcttt cgagtgggat 240
   actgtcaggt ttcacgcccc cgtacttgat gggaactccc gccttcacaa cccaccatgg 300
   ttaaaccccc ccggcctacg taagggtcat cagtcaagtt tagggcaatg ggtgttctgc 360
   attagttgcg tggtatcgta tgaaaccctt agttatcccg gg                    402
<210> 5
   <211> 3404
   <212> DNA
   <213> Turnip crinkle virus
<400> 5
   gggcgaattg ggcccgacgt cgcatgctcc cggccgccat ggccgcggga ttcccgggca 60
   ggcccccccc cgcgaggagg gaggctatct tttagttcgg aaggtcccca cagcccaccc 120
   tttcgggatt ttagtggtta cccaaagagc actagttttc cagtctaatg cccgcagcta 180
   gacagtgctg ccaccgtttt tggtccctaa cacaggtcaa aataaagcga cctgggggtt 240
   ttgtttcctt ttcttaatat aagagagagg agaagactct ttcgagtggg atactgtcag 300
   gtttcacgcc cccgtacttg atgggaactc ccgccttcac aacccaccat ggttaaaccc 360
   ccccggccta cgtaagggtc atcagtcaag tttagggcaa tgggtgttct gcattagttg 420
   cgtggtatcg tatgaaaccc ttagttatcc cgggaatcac tagtgcggcc gcctgcaggt 480
   cgaccatatg ggagagctcc caacgcgttg gatgcatagc ttgagtattc tatagtgtca 540
   cctaaatagc ttggcgtaat catggtcata gctgtttcct gtgtgaaatt gttatccgct 600
   cacaattcca cacaacatac gagccggaag cataaagtgt aaagcctggg gtgcctaatg 660
   agtgagctaa ctcacattaa ttgcgttgcg ctcactgccc gctttccagt cgggaaacct 720
   gtcgtgccag ctgcattaat gaatcggcca acgcgcgggg agaggcggtt tgcgtattgg 780
   gcgctcttcc gcttcctcgc tcactgactc gctgcgctcg gtcgttcggc tgcggcgagc 840
   ggtatcagct cactcaaagg cggtaatacg gttatccaca gaatcagggg ataacgcagg 900
   aaagaacatg tgagcaaaag gccagcaaaa ggccaggaac cgtaaaaagg ccgcgttgct 960
   ggcgtttttc cataggctcc gcccccctga cgagcatcac aaaaatcgac gctcaagtca 1020
   gaggtggcga aacccgacag gactataaag ataccaggcg tttccccctg gaagctccct 1080
   cgtgcgctct cctgttccga ccctgccgct taccggatac ctgtccgcct ttctcccttc 1140
   gggaagcgtg gcgctttctc atagctcacg ctgtaggtat ctcagttcgg tgtaggtcgt 1200
   tcgctccaag ctgggctgtg tgcacgaacc ccccgttcag cccgaccgct gcgccttatc 1260
   cggtaactat cgtcttgagt ccaacccggt aagacacgac ttatcgccac tggcagcagc 1320
   cactggtaac aggattagca gagcgaggta tgtaggcggt gctacagagt tcttgaagtg 1380
   gtggcctaac tacggctaca ctagaagaac agtatttggt atctgcgctc tgctgaagcc 1440
   agttaccttc ggaaaaagag ttggtagctc ttgatccggc aaacaaacca ccgctggtag 1500
   cggtggtttt tttgtttgca agcagcagat tacgcgcaga aaaaaaggat ctcaagaaga 1560
   tcctttgatc ttttctacgg ggtctgacgc tcagtggaac gaaaactcac gttaagggat 1620
   tttggtcatg agattatcaa aaaggatctt cacctagatc cttttaaatt aaaaatgaag 1680
   ttttaaatca atctaaagta tatatgagta aacttggtct gacagttacc aatgcttaat 1740
   cagtgaggca cctatctcag cgatctgtct atttcgttca tccatagttg cctgactccc 1800
   cgtcgtgtag ataactacga tacgggaggg cttaccatct ggccccagtg ctgcaatgat 1860
   accgcgagac ccacgctcac cggctccaga tttatcagca ataaaccagc cagccggaag 1920
   ggccgagcgc agaagtggtc ctgcaacttt atccgcctcc atccagtcta ttaattgttg 1980
   ccgggaagct agagtaagta gttcgccagt taatagtttg cgcaacgttg ttgccattgc 2040
   tacaggcatc gtggtgtcac gctcgtcgtt tggtatggct tcattcagct ccggttccca 2100
   acgatcaagg cgagttacat gatcccccat gttgtgcaaa aaagcggtta gctccttcgg 2160
   tcctccgatc gttgtcagaa gtaagttggc cgcagtgtta tcactcatgg ttatggcagc 2220
   actgcataat tctcttactg tcatgccatc cgtaagatgc ttttctgtga ctggtgagta 2280
   ctcaaccaag tcattctgag aatagtgtat gcggcgaccg agttgctctt gcccggcgtc 2340
   aatacgggat aataccgcgc cacatagcag aactttaaaa gtgctcatca ttggaaaacg 2400
   ttcttcgggg cgaaaactct caaggatctt accgctgttg agatccagtt cgatgtaacc 2460
   cactcgtgca cccaactgat cttcagcatc ttttactttc accagcgttt ctgggtgagc 2520
   aaaaacagga aggcaaaatg ccgcaaaaaa gggaataagg gcgacacgga aatgttgaat 2580
   actcatactc ttcctttttc aatattattg aagcatttat cagggttatt gtctcatgag 2640
   cggatacata tttgaatgta tttagaaaaa taaacaaata ggggttccgc gcacatttcc 2700
   ccgaaaagtg ccacctgatg cggtgtgaaa taccgcacag atgcgtaagg agaaaatacc 2760
   gcatcaggaa attgtaagcg ttaatatttt gttaaaattc gcgttaaatt tttgttaaat 2820
   cagctcattt tttaaccaat aggccgaaat cggcaaaatc ccttataaat caaaagaata 2880
   gaccgagata gggttgagtg ttgttccagt ttggaacaag agtccactat taaagaacgt 2940
   ggactccaac gtcaaagggc gaaaaaccgt ctatcagggc gatggcccac tacgtgaacc 3000
   atcaccctaa tcaagttttt tggggtcgag gtgccgtaaa gcactaaatc ggaaccctaa 3060
   agggagcccc cgatttagag cttgacgggg aaagccggcg aacgtggcga gaaaggaagg 3120
   gaagaaagcg aaaggagcgg gcgctagggc gctggcaagt gtagcggtca cgctgcgcgt 3180
   aaccaccaca cccgccgcgc ttaatgcgcc gctacagggc gcgtccattc gccattcagg 3240
   ctgcgcaact gttgggaagg gcgatcggtg cgggcctctt cgctattacg ccagctggcg 3300
   aaagggggat gtgctgcaag gcgattaagt tgggtaacgc cagggttttc ccagtcacga 3360
   cgttgtaaaa cgacggccag tgaattgtaa tacgactcac tata                  3404
<210> 6
   <211> 5451
   <212> DNA
   <213> Turnip crinkle virus
<400> 6
   gacgcgccct gtagcggcgc attaagcgcg gcgggtgtgg tggttacgcg cagcgtgacc 60
   gctacacttg ccagcgccct agcgcccgct cctttcgctt tcttcccttc ctttctcgcc 120
   acgttcgccg gctttccccg tcaagctcta aatcgggggc tccctttagg gttccgattt 180
   agtgctttac ggcacctcga ccccaaaaaa cttgattagg gtgatggttc acgtagtggg 240
   ccatcgccct gatagacggt ttttcgccct ttgacgttgg agtccacgtt ctttaatagt 300
   ggactcttgt tccaaactgg aacaacactc aaccctatct cggtctattc ttttgattta 360
   taagggattt tgccgatttc ggcctattgg ttaaaaaatg agctgattta acaaaaattt 420
   aacgcgaatt ttaacaaaat attaacgttt acaatttcag gtggcacttt tcggggaaat 480
   gtgcgcggaa cccctatttg tttatttttc taaatacatt caaatatgta tccgctcatg 540
   agacaataac cctgataaat gcttcaataa tattgaaaaa ggaagagtat gagtattcaa 600
   catttccgtg tcgcccttat tccctttttt gcggcatttt gccttcctgt ttttgctcac 660
   ccagaaacgc tggtgaaagt aaaagatgct gaagatcagt tgggtgcacg agtgggttac 720
   atcgaactgg atctcaacag cggtaagatc cttgagagtt ttcgccccga agaacgtttt 780
   ccaatgatga gcacttttaa agttctgcta tgtggcgcgg tattatcccg tattgacgcc 840
   gggcaagagc aactcggtcg ccgcatacac tattctcaga atgacttggt tgagtactca 900
   ccagtcacag aaaagcatct tacggatggc atgacagtaa gagaattatg cagtgctgcc 960
   ataaccatga gtgataacac tgcggccaac ttacttctga caacgatcgg aggaccgaag 1020
   gagctaaccg cttttttgca caacatgggg gatcatgtaa ctcgccttga tcgttgggaa 1080
   ccggagctga atgaagccat accaaacgac gagcgtgaca ccacgatgcc tgtagcaatg 1140
   gcaacaacgt tgcgcaaact attaactggc gaactactta ctctagcttc ccggcaacaa 1200
   ttaatagact ggatggaggc ggataaagtt gcaggaccac ttctgcgctc ggcccttccg 1260
   gctggctggt ttattgctga taaatctgga gccggtgagc gtgggtctcg cggtatcatt 1320
   gcagcactgg ggccagatgg taagccctcc cgtatcgtag ttatctacac gacggggagt 1380
   caggcaacta tggatgaacg aaatagacag atcgctgaga taggtgcctc actgattaag 1440
   cattggtaac tgtcagacca agtttactca tatatacttt agattgattt aaaacttcat 1500
   ttttaattta aaaggatcta ggtgaagatc ctttttgata atctcatgac caaaatccct 1560
   taacgtgagt tttcgttcca ctgagcgtca gaccccgtag aaaagatcaa aggatcttct 1620
   tgagatcctt tttttctgcg cgtaatctgc tgcttgcaaa caaaaaaacc accgctacca 1680
   gcggtggttt gtttgccgga tcaagagcta ccaactcttt ttccgaaggt aactggcttc 1740
   agcagagcgc agataccaaa tactgtcctt ctagtgtagc cgtagttagg ccaccacttc 1800
   aagaactctg tagcaccgcc tacatacctc gctctgctaa tcctgttacc agtggctgct 1860
   gccagtggcg ataagtcgtg tcttaccggg ttggactcaa gacgatagtt accggataag 1920
   gcgcagcggt cgggctgaac ggggggttcg tgcacacagc ccagcttgga gcgaacgacc 1980
   tacaccgaac tgagatacct acagcgtgag cattgagaaa gcgccacgct tcccgaaggg 2040
   agaaaggcgg acaggtatcc ggtaagcggc agggtcggaa caggagagcg cacgagggag 2100
   cttccagggg gaaacgcctg gtatctttat agtcctgtcg ggtttcgcca cctctgactt 2160
   gagcgtcgat ttttgtgatg ctcgtcaggg gggcggagcc tatggaaaaa cgccagcaac 2220
   gcggcctttt tacggttcct ggccttttgc tggccttttg ctcacatgtt ctttcctgcg 2280
   ttatcccctg attctgtgga taaccgtatt accgcctttg agtgagctga taccgctcgc 2340
   cgcagccgaa cgaccgagcg cagcgagtca gtgagcgagg aagcggaaga gcgcccaata 2400
   cgcaaaccgc ctctccccgc gcgttggccg attcattaat gcagagcttg caattcgcgc 2460
   gcgaaggcga agcggcattt acgttgacac catcgaatgg cgcaaaacct ttcgcggtat 2520
   ggcatgatag cgcccggaag agagtcaatt cagggtggtg aatgtgaaac cagtaacgtt 2580
   atacgatgtc gcagagtatg ccggtgtctc ttatcagacc gtttcccgcg tggtgaacca 2640
   ggccagccac gtttctgcga aaacgcggga aaaagtggaa gcggcgatgg cggagctgaa 2700
   ttacattccc aaccgcgtgg cacaacaact ggcgggcaaa cagtcgttgc tgattggcgt 2760
   tgccacctcc agtctggccc tgcacgcgcc gtcgcaaatt gtcgcggcga ttaaatctcg 2820
   cgccgatcaa ctgggtgcca gcgtggtggt gtcgatggta gaacgaagcg gcgtcgaagc 2880
   ctgtaaagcg gcggtgcaca atcttctcgc gcaacgggtc agtgggctga tcattaacta 2940
   tccgctggat gaccaggatg ccattgctgt ggaagctgcc tgcactaatg ttccggcgtt 3000
   atttcttgat gtctctgacc agacacccat caacagtatt attttctccc atgaagacgg 3060
   tacgcgactg ggcgtggagc atctggtcgc attgggtcac cagcaaatcg cgctgttagc 3120
   gggcccatta agttctgtct cggcgcgtct gcgtctggct ggctggcata aatatctcac 3180
   tcgcaatcaa attcagccga tagcggaacg ggaaggcgac tggagtgcca tgtccggttt 3240
   tcaacaaacc atgcaaatgc tgaatgaggg catcgttccc actgcgatgc tggttgccaa 3300
   cgatcagatg gcgctgggcg caatgcgcgc cattaccgag tccgggctgc gcgttggtgc 3360
   ggatatctcg gtagtgggat acgacgatac cgaagacagc tcatgttata tcccgccgtt 3420
   aaccaccatc aaacaggatt ttcgcctgct ggggcaaacc agcgtggacc gcttgctgca 3480
   actctctcag ggccaggcgg tgaagggcaa tcagctgttg cccgtctcac tggtgaaaag 3540
   aaaaaccacc ctggcgccca atacgcaaac cgcctctccc cgcgcgttgg ccgattcatt 3600
   aatgcagctg gcacgacagg tttcccgact ggaaagcggg cagtgagcgc aacgcaatta 3660
   atgtgagtta gctcactcat taggcacccc aggctttaca ctttatgctt ccggctcgta 3720
   tgttgtgtgg aattgtgagc ggataacaat ttcacacagg aaacagctat gaccatgatt 3780
   acgccaagct ctaatacgac tcactatagg gaaagctggt acgcctgcag gtaccggtcc 3840
   ggaattggga taactaaggg tttcatacga taccacgcaa ctaatgcaga acacccattg 3900
   ccctaaactt gactgatgac ccttacgtag gccggggggg tttaaccatg gtgggttgtg 3960
   aaggcgggag ttcccatcaa gtacgggggc gtgaaacctg acagtatccc actcgaaaga 4020
   gtcttctcct ctctcttata ttaagaaaag gaaacaaaac ccccaggtcg ctttattttg 4080
   acctgtgtta gggaccaaaa acggtggcag cactgtctag ctgcgggcat tagactggaa 4140
   aactagtgct ctttgggtaa cctagagtcg cggccgcttt acttgtacag ctcgtccatg 4200
   ccgagagtga tcccggcggc ggtcacgaac tccagcagga ccatgtgatc gcgcttctcg 4260
   ttggggtctt tgctcagggc ggactgggtg ctcaggtagt ggttgtcggg cagcagcacg 4320
   gggccgtcgc cgatgggggt gttctgctgg tagtggtcgg cgagctgcac gctgccgtcc 4380
   tcgatgttgt ggcggatctt gaagttcacc ttgatgccgt tcttctgctt gtcggccatg 4440
   atatagacgt tgtggctgtt gtagttgtac tccagcttgt gccccaggat gttgccgtcc 4500
   tccttgaagt cgatgccctt cagctcgatg cggttcacca gggtgtcgcc ctcgaacttc 4560
   acctcggcgc gggtcttgta gttgccgtcg tccttgaaga agatggtgcg ctcctggacg 4620
   tagccttcgg gcatggcgga cttgaagaag tcgtgctgct tcatgtggtc ggggtagcgg 4680
   ctgaagcact gcacgccgta ggtcagggtg gtcacgaggg tgggccaggg cacgggcagc 4740
   ttgccggtgg tgcagatgaa cttcagggtc agcttgccgt aggtggcatc gccctcgccc 4800
   tcgccggaca cgctgaactt gtggccgttt acgtcgccgt ccagctcgac caggatgggc 4860
   accaccccgg tgaacagctc ctcgcccttg ctcaccatgg taaccactaa aatcccgaaa 4920
   gggtgggctg tggggacctt ccgaactaaa agatagcctc cctcctcgcg ggggggggcc 4980
   tgcccgggtc ggcatggcat ctccacctcc tcgcggtccg acctgggcat ccgaaggagg 5040
   acgcacgtcc actcggatgg ctaagggagg ggcggggatc cggctgctaa caaagcccga 5100
   aaggaagctg agttggctgc tgccaccgct gagcaataac tagcataacc ccttggggcc 5160
   tctaaacggg tcttgagggg ttttttgctg aaaggaggaa ctatatccgg atccactagt 5220
   tctagaggat ccaagcttac gtacgcgtgc atgcgacgtc atagctcttc tatagtgtca 5280
   cctaaattca attcactggc cgtcgtttta caacgtcgtg actgggaaaa ccctggcgtt 5340
   acccaactta atcgccttgc agcacatccc cctttcgcca gctggcgtaa tagcgaagag 5400
   gcccgcaccg atcgcccttc ccaacagttg cgcagcctga atggcgaatg g          5451

## Beschreibung

Die Erfindung betrifft eine neuartiges Verfahren zur konstitutiven oder induzierbaren, stabilen oder transienten intrazellulären Amplifikation von Fremd-RNA in tierischen Zellen. Das Verfahren beruht auf einer autonomen, RNA-abhängigen RNA-Amplifikation durch Expression der RNA-abhängigen RNA-Polymerase (RdRP) eines Pflanzenvirus in tierischen Zellen.

Initiation der Amplifikation erfolgt durch ein RNA-Transkript (Primärtranskript) mit den Promotoren oder cis-aktiven Sequenzen für diese RdRP (Abkürzungsverzeichnis hinter den Beispielen). Die amplifizierte RNA kann als mRNA zur Proteinsynthese dienen, als Effektor-RNA (zum Beispiel als anti-sense-RNA gegen bestimmte mRNA oder virale RNA-Moleküle, als Ribozym gegen zelluläre- RNA-Moleküle oder rekombinante Struktur-RNA in Ribosomen oder Spliceosomen) oder als genomische RNA für die Herstellung rekombinanter Viren. Die Erfindung schließt ein Medikament zur Gentherapie, Vakzinierung oder therapeutische Vakzinierung ein.

### STAND DER TECHNIK

Zur Expression von Fremdgenen in tierischen Zellen werden meist die offenen Leseraster als cDNA (complementary DNA, copy DNA) in die Zelle transfiziert. Diese cDNA kann in Plasmide eingefügt und von cis-aktiven Sequenzen wie Promotoren und Polyadenylierungssignalen flankiert sein, die eine Synthese von mRNA (Messenger-Ribonucleinsäure, Boten-RNA) in der Zielzelle erlauben.

Die Transfektion von Plasmid-DNA in eine Wirtszelle führt in der Regel nur zu einer vorübergehenden oder transienten Expression von Fremdgenen: Mit der Teilung der transfizierten Zellen und durch Degradation der eingebrachten DNA stehen den Tochterzellen immer weniger Plasmide zur Verfügung und gehen innerhalb weniger Wochen entgültig verloren [Ausubel et al. (ed) 2000, Current Protocols in Molecular Biology, John Wiley & Sons].

Für eine stabile Expression muss das Fremdgen somit entweder in das Chromosom der Wirtszelle integriert, oder aber, vermittelt durch Funktionen im Plasmidrückgrat, in seiner Replikation episomal an die Zellteilung gekoppelt werden. Ist das Fremdgen toxisch für die Zelle, dann muss der Promoter während der Etablierung der Zelllinie blockiert werden. Für eine induzierbare Stummschaltung von Promotoren stehen mehrere Systeme zur Verfügung. Allen Systemen ist jedoch eine gewisse basale Aktivität des kontrollierten Promotors gemein, so dass es zu einer messbaren Hintergrundexpression der mRNA kommen kann [Ausubel et al. (ed) 2000, Current Protocols in Molecular Biology, John Wiley & Sons].

Für eine hohe Expression von rekombinanten Fremdgenen werden bislang hauptsächlich drei Parameter variiert: (1) die Stärke des Promoters, der die Synthese der mRNA-Transkripte bewirkt; (2) die Translatierbarkeit der resultierenden mRNA vor allem über Optimierung von RNA-Export in das Zytoplasma, der Initiator tRNA-Umgebung (tRNA - Transport-RNA) und Codonfrequenz im offenen Leseraster; und (3) die Zahl der DNA-Kopien durch eine einmalige chemisch-induzierte Gen-Amplifikation integrierter Kopien, oder der Zahl von Plasmidmolekülen über SV40-vermittelte oder episomale Anreicherung. Optimierung der ersten beiden Parameter ist beim heutigen Stand der Technik weit vorangeschritten und scheint die Leistungsfähigkeit herkömmlicher Wirtszellen weitgehend auszuschöpfen. Die Zahl der DNA-Kopien hat ab einem bestimmten Schwellenwert nur noch geringen Zuwachs in der Ausbeute zur Folge und ist außerdem im Fall der Amplifikation integrierter DNA aufgrund der ungezielten Manipulation im Genotyp der Wirtszelle in ihren Auswirkungen kaum vorhersagbar [Snapka et al. 1997, Cell. Prolif. 30, 385-99].

In anderen Ansätzen werden rekombinante Viren verwendet, die eine Expression der cDNA in hoher Menge bei oftmals optimalen Transduktionseffizienzen ermöglichen. Etablierte Systeme nutzen vor allem Vakzinia-, Herpes-, Adeno-, Alpha-, Retro- und Parvoviren (Adeno-assozierte Viren); neuere Entwicklungen versuchen den Einsatz von Polio-, Flavi- und Rhabdoviren (Tollwutviren). In einigen Ansätzen unterbricht das Fremdgen einen für die virale Replikation in der Zellkultur unerheblichen Bereich, so dass replikationskompetente, transgene
Viren entstehen. Das Fremdgen kann jedoch auch einen für die Replikation oder Morphogenese wichtigen Bereich ersetzen. Derartige Viren exprimieren das Fremdgen, können jedoch ohne Transkomplementierung der fehlenden Faktoren keine Nachfolgeviren bilden.
Gegenüber der Transfektion von Expressionsplasmiden liegt der Vorteil viraler Systeme vor allem in höherer Transduktionseffizienz und - im Falle replikationskompetenter Systeme - höherer Expressionsstärke aufgrund Virusmultiplikation und Hemmung der Wirtszellbiosynthese, so dass mehr Ressourcen für die virale Expression zur Verfügung stehen. Allerdings ist eine stabile Transduktion in diesem Fall nicht möglich, weil die Hochexpression des Fremdgens an eine produktive Infektion gekoppelt ist. Obgleich es für einige Systeme - zum Beispiel für Vakziniaviren, Herpesviren, Adenoviren und Alphaviren - Virusmutanten mit attenuierter Zytopathogenizität gibt, führt die Aktivierung des Virus in der Regel zum Tod der Wirtszelle. Darüber hinaus sind derartige Präparationen mit der Gefahr einer Verunreinigung durch Helferviren oder der Entstehung von Rekombinanten mit unbekannten Eigenschaften behaftet. Dieser Aspekt ist wichtig, da die oben genannten Systeme in der Regel von Humanpathogenen abgeleitet sind. Akzidentale Laborinfektionen durch die Arbeit mit verschiedenen Systemen und der Tod eines Laborarbeiters durch Semliki-Forest-Virus (ein Alphavirus) sind beschrieben worden [Willems et al. 1979, Science 203, 1127-9]. Auch attenuierte Viren können in immunosupprimierten Patienten ein höheres pathogenes Potential entwickeln [Lustig et al., 1999, Arch Virol 144, 1159-71; Centers for Disease Control and Prevention 2001. MMWR 50 NO. RR-10].
Bei Viren mit einer DNA-Phase in der Replikation ist außerdem die Gefahr der Integration von Viren in das Genom der Zelle gegeben; vor allem bei Retroviren ist Integration sogar ein Bestandteil der Virusreplikation. Da eine Integration den Phenotyp der Wirtszelle ändern kann, in der Gentherapie möglicherweise sogar zur Transformation und damit Krebs führen kann, ist sie in manchen Ansätzen eine unerwünschte Begleiterscheinung.
Gegenüber einer Transfektion von Expressionsplasmiden sind viele virale Systeme methodisch deutlich anspruchsvoller in der Herstellung der Vektoren. Vakzinia-, Adeno- und Herpesviren sind aufgrund der großen Genome einer direkten Manipulation nur schwer zugänglich und erfordern Rekombinationsereignisse zwischen Empfänger- und Transfervektoren.
Außerdem ist auch in den weniger komplexen Systemen wie Flavi- und Alphavirusvektoren die Expression des Fremdgens an die Ausbildung von heteromeren viralen Proteinkomplexen und an Replikationsereignisse gebunden, die bislang unvollständig verstanden sind [Khromykh et al., 1999, J Virol 73, 10272-80; Perri et al. 2000, J Virol 74, 9802-7].
In einem weiteren Ansatz werden über rekombinante T7-RNA-Polymerase im Zytoplasma unmodifizierte RNA-Moleküle synthetisiert (weder mit Cap-Struktur versehen noch polyadenyliert). Für eine effiziente Translation müssen diese Transkripte mit IRES-Elementen (IRES - internal ribosomal entry site) versehen werden [Elroy-Stein, 1989, PNAS 86, 6126-30]. Als Matritze dient in der Regel Plasmid-DNA, die sich nach einer Transfektion auf dem Weg zum Zellkern jedoch nur kurzfristig im Zytoplasma befindet. Über künstlich eingeführte Kernlokalisationsequenzen konnte die T7-RNA-Polymerase in das gleiche zelluläre Kompartiment gelenkt werden, in dem diese Matrizen akkumulieren. Allerdings führten diese Ansätze zu ineffizienter Proteinexpression, da vermutlich die synthetisierte RNA nicht in den Exportweg für mRNA-Moleküle gelenkt wird und somit nur in geringer Zahl das Zytoplasma, Ort der Proteinbiosynthese, erreicht [Lieber et al. 1993, Meth. Enzym. 217, 47-66].

### BESCHREIBUNG DER ERFINDUNG

Der Erfindung liegt die Aufgabe zugrunde, ein Verfahren zu entwickeln, mit dem eine Amplifikation von Nukleinsäuren in tierischen Zellen ermöglicht wird.
Die Aufgabe wurde durch ein Verfahren gelöst, das das Einbringen
(i) einer RNA-abhängigen RNA-Polymerase (RdRP) eines Pflanzenvirus und
(ii) einer RNA, die Promotoren oder cis-aktive Signale enthält, welche von der RdRP erkannt werden (Substrat-RNA)
in tierische Zellen umfasst. Dieses Verfahren zur Amplifikation von RNA in tierischen Zellen, außer humanen Stammzellen, aber einschließlich von Säuger-, auch Human-, Insekten-, Wurm- und Amphibienzellen, beruht darauf, dass die RdRP eines Pflanzenvirus in tierische Zellen zusammen mit einer Substrat-RNA eingebracht wird, die ein oder mehrere Promotoren oder cis-aktive Signale enthält, welche von der RdRP erkannt werden. Das Gen der RdRP kann aus Pflanzenzellen oder einem Pflanzenvirus, bevorzugt aus der Familie der Tombusviridae, gewonnen werden. Die Substrat-RNA kann als Primärtranskript in der Wirtszelle synthetisiert oder in die tierische Zelle von außen eingebracht werden.
Die Erfindung betrifft des Weiteren eine tierische Zelle mit Ausnahme humaner embryonaler Stammzellen, enthaltend
(i) eine RNA-abhängigen RNA-Polymerase (RdRP) eines Pflanzenvirus und
(ii) eine RNA, die Promotoren oder cis-aktive Signale enthält, welche von der RdRP erkannt werden (Substrat-RNA). Zudem betrifft sie Isolierte Transkripte, die zum Einbringen

(i) einer RNA-abhängigen RNA-Polymerase (RdRP) eines Pflanzenvirus und
(ii) einer RNA die Promotoren oder cis-aktive Signale enthält, weiche von der RdRP erkannt werden (Substrat-RNA), in tierische Zellen mit Ausnahme humaner embryonaler Stammzellen geeignet sind und
   (a) welche eine RdRP codieren; und
   (b) welche ein Fremdgen und zumindest einen Promoter, der durch RdRP erkannt wird, enthalten, wobei beide in Antisense-Orientierung vorliegen und das Fremdgen ohne die RdRP nicht exprimiert werden kann, aber durch deren Expression aktiviert wird.
Diese Transkripte werden in einem weitern Aspekt der Erfindung zur in-vitro-Amplifikation von Nukleinsäuren, insbesondere zur Amplifikation von RNA in tierischen Zellen mit Ausnahme humaner embryonaler Stammzellen verwendet. Schließlich umfasst die Erfindung auch ein Medikament zur Gentherapie, Vakzinierung oder therapeutischen Vakzinierung enthaltend ein solches Transkript.

Das erfindungsgemäße Verfahren zur Amplifikation von
Nukleinsäuren in tierischen Zellen umfasst das Einbringen einer RNA-abhängigen RNA-Polymerase (RdRP) eines Pflanzenvirus in die tierischen Zellen.
Wesentliche neuartige Merkmale des beschriebenen Verfahrens sind (1) Expression eines funktionellen pflanzlichen Replikons in einer tierischen Zelle; (2) ein zweifacher Block zur Stummschaltung von Genen, da das Primärtranskript Gene in nicht-kodierender Orientierung tragen kann und die Umsetzung in eine kodierende mRNA nur in der Gegenwart der, ebenfalls regulierbaren, RdRP erfolgt; (3) Reduktion der RNA-abhängigen RNA-Amplifikation auf eine Polymerase und das damit verwandte Primärtranskript. Virale Strukturgene oder Helferviren werden nicht benötigt. (4) Polymerase und Substrat-RNA können unabhängig voneinander exprimiert werden.
Mit der erfindungsgemäßen Lösung gelingt es überraschenderweise, eine Amplifikation von Nukleinsäuren in tierischen Zellen über eine möglichst geringe Zahl von Elementen bevorzugt direkt im Cytoplasma und frei von für Tiere potentiell infektiösen Komponenten zu erreichen.
Die Erfindung unterscheidet sich damit von Patentenanmeldungen wie zum Beispiel WO 99/02718 A1 darin, dass ein pflanzenvirales System in überraschend reduzierter Weise auf tierische Modelle übertragen wird. Die Erfindung unterscheidet sich außerdem von Patentenanmeldungen, die den Einsatz isolierter Polymerasen oder RdRPs zur Amplifikation von Nukleinsäuren beschreiben (zum Beispiel WO 99/49085 A1 und US 5556769) darin, dass hier ein derartiges Amplifkationssystem in der lebenden Zelle (und nicht in Zellextrakten) verwirklicht wird, womit die vorliegende Erfindung auch in Bioreaktoren zum Einsatz kommen kann und zum Beispiel die amplifizierte RNA nicht nur in ein Protein oder in mehrere Proteine translatiert werden kann, sondern auch post-translationelle Modifikationen wie Phosphorylierung, Glykosylierung oder proteolytische Reifung ermöglicht werden.
Das erfindungsgemäße Verfahren beruht in einer bevorzugten Ausführungsform auf der Adaption der Replikation des Pflanzenvirus TCV (Turnip-Crinkle-Virus; *Tombusviridae)* in der tierischen Zelle. Das Verfahren erweitert das Potential herkömmlicher Denkansätze durch den ungewöhnlichen Einsatz pflanzenviraler Enzyme in Säugerzellen. Es kann durchaus mit anderen Viren der *Tombusviridae* verwirklicht werden, zum Beispiel Carnation-Mottle-Virus, oder auch mit anderen Virusfamilien mit ähnlicher genomischer Organisation, wie zum Beispiel den *Bromoviridae,* oder gar segmentierten Viren wie den *Potyviridae* [Mayo & Pringle, 1998 in J. Gen. Virol. 79, 649-57]. Ein weiterer gewünschter, äußerst wichtiger Vorteil gegenüber allen herkömmlichen Verfahren ist die Tatsache, dass es unter den *Tombusviridae* und einigen anderen Pflanzenvirusfamilien keine Pathogene für Menschen und andere Wirbeltiere gibt.
Während der Bereitung dieses Manuskripts wurde die Biosynthese einer pflanzlichen RdRP zur Aufreinigung auch in Säugerzellen vorgeschlagen (Patent US 6218142 B1 2001). Hierbei ist jedoch nicht die RNA-Amplifikation und Fremdgenexpression durch eine rekombinante RdRP beabsichtigt, sondern die Inaktivierung oder Überexpression einer endogenen RdRP, bevorzugt aus uninfizierten Tomaten, die in pflanzlichen Zellen, und möglicherweise auch Säugerzellen, über den Mechanismus des *Gene-Silencing* die Expression fremder Gene inhibieren kann. Des weiteren ist die Expression einer rekombinanten RdRP in Fremdzellen zur Vermittelung von Gene-Silencing beabsichtigt. In der vorliegenden Anmeldung soll jedoch eine pflanzenvirale RdRP, die nicht mit der RdRP in der Schrift US 6218142 B1 2001 verwandt ist, bevorzugt zur Hochexpression kodierender RNA eingesetzt werden.
Das Genom der Viren der Familie der Tombusviridae besteht aus einem einzelnen RNA-Strang von etwa 4500 Nukleotiden in kodierender Orientierung. Für das Carnation-Mottle-Virus dieser Familie wurde am 5'-Ende der genomischen RNA eine Cap-Struktur nachgewiesen. Die Replikation der Tombusviridae verlangt keine Wirtszellfaktoren oder post-translationelle Modifikation an den viralen Proteinen. Im deutlichen Gegensatz zu verwandten alphaviralen Systemen liegen die Promotorelemente an den Termini der genomischen RNA; die interne Initiation der Transkription zur Synthese subgenomischer RNA-Moleküle ist nicht notwendig.
Für die hier vorgestellte Anwendung wird als virales Protein die RNA-abhängige RNA-Polymerase (RdRP) rekombinant exprimiert, also im Gegensatz zu einigen anderen Systemen ohne die Notwendigkeit der Komplementation durch ein Helfervirus. Als weiterer Vorteil gegenüber adeno- oder polioviralen Systemen ist eine kovalente Modifikation der genomischen Nukleinsäure durch terminale Proteine nicht notwendig. Als Edukt zur intrazellulären Amplifikation von RNA dient ein Primärtranskript, das die entsprechenden erkennbaren cis-aktiven Signale - zum Beispiel virale Promotoren (VP) oder artifizielle Promotoren oder Terminatoren - von wenigen hundert Nukleotiden trägt. Neuartig ist weiterhin, dass virale Gene auf dem Primärtranskript nicht benötigt werden. Auch Verpackung in virale Kapside ist keine Voraussetzung für Amplifikation, so dass das Primärtranskript sehr große Fremdgene oder Insertionen akzeptieren kann.

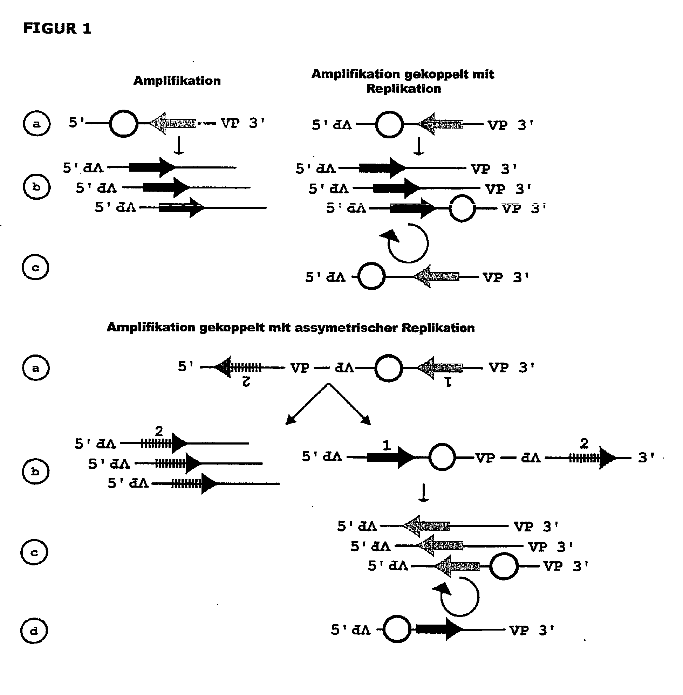

*Tombusviridae* verfügen über verschiedene Promotoren mit unterschiedlichen Expressionsstärken. Da Substrat-RNA und RdRP unabhängig voneinander exprimiert werden können, ist es außerdem im Gegensatz zu bislang bekannten Verfahren möglich, über mehrere verschiedene Substrat-RNAs Gruppen von Genen gemeinsam zu exprimieren. Dieser modulare Aufbau erlaubt eine neuartige und überraschende Flexibilität in der Durchführung von Expression, Ko-Expression oder Hochexpression von Proteinen sowie einseitiger Amplifikation oder Replikation von RNA (auch mit Cap-Struktur versehener mRNA). Trotz der hohen Flexibilität ist die Herstellung der Expressionskassetten über direkte, unkomplizierte, dem Fachmann vertraute Methoden zu erreichen und erfordert keine Rekombination oder Komplementierung durch Helferviren.
In einer bevorzugten Anwendung wird als Substrat-RNA ein Primärtranskript direkt in der Zelle, entweder im Zytoplasma oder im Nukleus, von zellulären RNA-Polymerasen wie zum Beispiel RNA-Polymerase II oder von rekombinanten Polymerasen wie RNA-Polymerase T7 synthetisiert. Alternativ kann die Substrat-RNA aber auch *in vitro,* zum Beispiel mit der Bakteriophagen T7-, Sp6- oder T3-RNA-Polymerase synthetisiert und in die Zelle transfiziert werden.
In einer bevorzugten Ausführung des Verfahrens wird die RdRP in trans entweder durch Induktion oder konstitutive Expression über eine eigene Expressionskassette zur Verfügung gestellt. Die Substrat-RNA wird unabhängig von der RdRP exprimiert. Auf der Substrat-RNA folgt ein VP einem Fremdgen. Sowohl Fremdgen als auch VP liegen in nicht-kodierender Orientierung vor (siehe Figur 1). Auf diese Weise wird gewährleistet, dass das Fremdgen nicht translatiert werden kann, selbst wenn die Stummschaltung der Expressionskassette für die Substrat-RNA nicht vollständig sein sollte. Somit ist eine weitere Sicherung in der Expression toxischer Genprodukte eingebaut. Ein derartiger Ansatz zur Kontrolle der Genexpression ist unseres Wissens nach bislang noch nicht beschrieben worden. Die Substrat-RNA dient der RdRP als Substrat für die Synthese einer anti-sense-Kopie. Diese Kopie jedoch trägt das Fremdgen in kodierender Orientierung, so dass es nun zur Translation kommen kann. Da die RdRP der *Tombusviridae* ihre Transkripte wahrscheinlich mit einer Cap-Struktur versieht, ist zur Translation kein IRES-Element notwendig. In weiteren Anwendungen kann jedoch die Translationseffizienz durch eine vorgeschaltete IRES verändert oder regulierbar gemacht werden. Der Schritt der Substrat-RNA-Erkennung durch die virale RdRP führt zur RNA-abhängigen Amplifikation einer mRNA und damit zur Hochexpression eines Fremdgens. Ein großer neuartiger Vorteil dieses Systems gegenüber herkömmlichen viralen Expressionsmethoden ist Regulierbarkeit: Wird die Expression der RdRP oder der Substrat-RNA blockiert, so kommt das System zum Erliegen.
In einer weiteren Ausführung flankieren die VPs das Fremdgen (siehe Figur 1). Eine derartige Substrat-RNA wird repliziert und amplifiziert: Die kodierenden Transkripte können nun als Matrize für die Synthese weiterer Kopien des Primärtranskripts dienen. Diese Kopien wiederum bringen weitere kodierende Transkripte hervor, womit eine exponentielle Steigerung der Anzahl spezifischer Transkripte als Voraussetzung für deutlich gesteigerte Fremdgenexpression erreicht wird. Auch hier kann das System im deutlichen Gegensatz zu herkömmlichen, persistierenden viralen Systemen trotz höchster Expressionsraten für das Fremdgen durch einen Block in der Expression der RdRP ausgeschaltet werden. In weiteren Ausführungen dieses Verfahrens kann als Substrat-RNA natürliche oder modifizierte Satelliten-RNA verwendet werden. In wieder anderen Ausführungen kann durch geeignete Wahl wildtypischer Promotoren (zum Beispiel Promotoren in Satelliten-RNA [Carpenter & Simon 1998 in Nucl. Acids Res. 26, 2426-32 und Simon et al. 1988 in EMBO J 7, 2645-51], oder auf der genomischen RNA [Carrington et al. 1989 in Virology 170, 219-26]) oder durch Mutagenese von cis-aktiven Sequenzen der VP für die nichtkodierenden Transkripte deutlich schwächer oder stärker eingestellt werden als der VP, der die kodierenden Transkripte hervorbringt. Dadurch ist eine weitere Modulation der Expressionseffizienz möglich.
In einer weiteren Ausführung flankieren die VPs in unabhängigen Transkripten sowohl RdRP als auch das Fremdgen. Bei diesen Verfahren wird die Expression sowohl der RdRP als auch des Fremdgens amplifiziert und repliziert. Je nach Wahl und Stärke der VPs durch den Experimentator kann das Verfahren drei Wege gehen: (1) die Wirtszelle erliegt der Hochexpression und stirbt. (2) Ein künstliches, nur auf RNA basierendes Replikon entsteht und wird in der Zelle erhalten. (3) Die Replikation sinkt langsam ab, falls der Turnover der RNA und RdRP nicht durch die Replikationsstärke in den VPs kompensiert werden kann.
In Aspekten der oben genannten Ausführung kodiert die Substrat-RNA für die RdRP und ein Fremdprotein und enthält außerdem funktionelle virale Promotoren an beiden Termini. Diese Substrat-RNA wird als RNA in kodierender Orientierung bezüglich der RdRP in die Zelle oder einen Organismus eingebracht und initiiert so die Replikation. Auf diese Weise wird die Transduktion von Zellen unter Umgehung einer DNA-Phase erreicht, womit die Gefahr von unbeabsichtigten Modifikationen im Genom der Wirtszelle vermindert wird. Da die Proteinexpression und Replikation im Zytoplasma erfolgt, eine Kernlokalisation somit nicht benötigt wird, sind auch ruhende Zellen durch dieses System transduzierbar.
In einer weiteren Ausführung steht anstelle des Fremdgens ein RNA-Abschnitt, der ohne Translation eine Wirkung in der Zelle entfaltet, wie zum Beispiel Ribozyme, ribosomale RNA oder Antisense-Konstrukte oder eine genomische oder subgenomische RNA eines anderen Virus. Somit kann diese funktionelle RNA durch das Verfahren amplifiziert und deren Wirkung in der Zelle verstärkt werden. Im Falle des Einsatzes einer genomischen oder subgenomischen RNA eines anderen Virus werden Effekte der Virusinfektion simuliert, ohne die Replikationsmaschinerie des jeweiligen Virus zu verwenden. Dabei entsteht ein besonders sicheres, stark attenuiertes System. Alternativ wird das Verfahren zur Herstellung rekombinanter Viren verwendet.
In Aspekten der oben genannten Ausführungen wird die Aktivität der RdRP durch Ausnützung des Temperaturoptimums moduliert, indem die Kulturtemperatur der Säugerzellen entsprechend verändert wird.
In weiteren Aspekten dieses Verfahrens werden Substrat-RNAs derart verändert, dass eine verbesserte oder weniger gute Substraterkennung durch die RdRP erfolgt. Methoden dazu können Mutagenese in cis-aktiven Sequenzen, Insertion und Deletion beinhalten. Des weiteren kann die Größe des Primärtranskripts variiert werden. Durch die Wahl verschiedener oder gleichartiger Promotoren oder von Promotoren und Satelliten-RNAs verwandter Viren können Transkripte mit unterschiedlichen Replikationseigenschaften generiert werden. Interne Promotoren auf den Primärtranskripten können zur Synthese von kleineren abgeleiteten Transkripten eingesetzt werden. Die Position und Zahl der Fremdgene kann variiert werden. Die Zahl verschiedener Primärtranskripte kann variiert werden.
In weiteren Aspekten wird die Translatierbarkeit und Halbwertszeit der entstehenden Transkripte über künstliche Polyadenylylabschnitte oder durch das Einfügen von Elementen verbessert, welche die Wechselwirkung mit Ribosomen verändern, wie zum Beispiel Stamm-Schleifen, IRES-Elemente wie die von Encephalomyocarditis-Virus oder Poliovirus [Pestova et al. 2001 in Proc. Natl. Acad. Sci. USA 98, 7029-36], Shunt-Donor und -Akzeptor wie die von Cauliflower-Mosaic-Virus [Fütterer et al. 1993 in Cell 73, 789-802] oder Translationsenhancer wie der Leader vor dem zellulären Protein p27 [Miskimins et al. 2001 in Mol. Cell. Biol. 21, 4960-7].
In weiteren Aspekten des Verfahrens wird die von der RdRP amplifizierte RNA mit Erkennungssignalen ausgestattet, die eine Verpackung durch virale Kapsidproteine oder ssRNA-bindende Proteine (ssRNA binding proteins) bewirken können, oder die eine Prozessierung der Transkripte durch Endonukleasen, RNA-Editing Enzyme oder durch die Spleißmaschinerie ermöglichen. In verwandten Aspekten können die Erkennungssignale derart gestaltet werden, dass nur die amplifizierten Transkripte einer bestimmten Orientierung erkannt werden.
In einer bevorzugten Anwendung des Verfahrens wird die höhere intrinsische Fehlerrate einer RNA-abhängigen RNA-Amplifikation zur breitgestreuten Mutagenese und Auffindung besonders geeigneter Mutationen in RNA-Abschnitten genutzt, die für ein Gen kodieren können oder aber für Bereiche stehen, die als untranslatierte RNA ihre Wirkung entfalten, wie zum Beispiel die viralen Promotoren des Systems selbst, Ribozyme, anti-sense-RNA oder Struktur-RNA in Ribosomen oder Spliceosomen. In einer besonders attraktiven Anwendung des Systems können die amplifizierten Transkripte für ein Fremdgen kodieren, dessen Signalstärke im Zeitverlauf verfolgt wird, bis besonders geeignete Mutanten im Fremdgen oder in den viralen Promotoren gefunden sind. Da die flankierenden Sequenzen bekannt sind, kann die entsprechende Sequenz sehr einfach gezielt über PCR gewonnen werden. In einer weiteren Anwendung des Verfahrens kann das Fremdgen durch ein Protein mit unerwünschter Zytotoxizität ersetzt werden; in einer Zeitstudie können Replikons, deren Wirtszellen besser als benachbarte Zellen wachsen, zur Auffindung von Mutanten mit besonders geringer Zytotoxizität gerettet werden.
In einer weiteren bevorzugten Anwendung des Verfahrens kann die RdRP mit einer Erkennungssequenz für ein regulierbares Protein gekoppelt werden, wie zum Beispiel Inhibitor-kappa-B (IkB) oder ein modifizierter Östrogenrezeptor. Bindung durch das zelluläre Protein führt zum reversiblen Block der RdRP-Aktivität, der
mit der Dissoziation des Faktors aufgehoben wird. Die aktive RdRP könnte zum Beispiel die Transkription der anti-sense-RNA für ein Fremdgen bewirken, das nun translatiert werden kann. Auf diese Weise kann ein intrazelluläres Messsystem für bestimmte zelluläre Wechselwirkungen aufgebaut werden.
Die Merkmale der Erfindung gehen aus den Elementen der Ansprüche und aus der Beschreibung hervor. Die Merkmale setzen sich aus neuen - dem Einsatz pflanzenviraler Enzyme (der RNA-abhängigen RNA-Polymerase (RdRP)) in tierischen Zellen sowie der Trennung von Transkripten für RdRP (mit Aktivität einer Replikase) und Substrat-RNA - und bekannten Elementen - RNA-Amplifikation und Proteinsynthese - zusammen, die in ihrer Kombination zu dem erfindungsgemäßen neuen vorteilhaften Verfahren führen.
Die Erfindung besteht in einem Verfahren zur Amplifikation von RNA in tierischen Zellen unter Verwendung einer Substrat-RNA-abhängigen RNA-Polymerase (RdRP) eines Pflanzenvirus und einer Substrat-RNA, die Promotoren oder cis-aktive Signale enthält, welche von der RdRP erkannt werden. Das erfindungsgemäße Verfahren bezieht sich auf tierische Zellen, einschließlich von Säuger-, auch Human- (mit Ausnahme embryonaler Stammzellen), Insekten-, Wurm-, und Amphibienzellen. Es umfasst das Einbringen einer RdRP eines Pflanzenvirus, deren Gen aus Pflanzenzellen oder einem Pflanzenvirus gewonnen und in tierische Zellen eingebracht wird, und RNA, die einen Promotor oder mehrere Promotoren oder cis-aktive Signale, welche von der RdRP erkannt werden, und die als Primärtranskript synthetisiert oder in die tierische Zelle von außen eingebracht wird in tierischen Zellen.
Das erfindungsgemäße Verfahren beruht bevorzugt darauf, dass die RdRP durch ein RdRP-Gen, das in die tierischen Zellen eingebracht wird, codiert wird.

Das erfindungsgemäße Verfahren verwendet in bevorzugten Ausführungsformen zur intrazellulären Amplifikation von RNA eine RdRP,
- die von einem gesonderten Transkript getrennt von ihrem Promotor kodiert wird oder
- deren Gen Teil der Substrat-RNA ist, oder
- deren Gen Teil ihres eigenen Transkripts ist,
   sowie eine Substrat-RNA,
- deren Eigenschaften als Substrat durch Mutagenese verändert werden, und/oder
- deren Fremdgen und zumindest ein Promotor in antisense-Orientierung vorliegen, so dass das Fremdgen ohne RdRP nicht exprimiert werden kann, und/oder
- die IRES-Elemente, Shunt-Donor und -Akzeptor oder Translationsenhancer zur Verbesserung der Genexpression trägt,
wobei die amplifizierte RNA
- mindestens einen Polyadenylyltrakt enthält, und/oder
- durch ein Ribozym prozessiert wird, und/oder
- Signale zur Verpackung in eine Virushülle enthält und in Virushüllen verpackt wird, und/oder
- für ein Gen oder mehrere Gene kodiert, und/oder
- für Gene in unterschiedlichen Orientierungen kodiert, und/oder
- für eine RNA steht, die ohne Translation eine Wirkung in der Zelle entfaltet, wie zum Beispiel Ribozyme, ribosomale RNA oder Antisense-Konstrukte, und/oder
- für eine genomische oder subgenomische RNA eines anderen Virus kodiert, und/oder verwendet eine RdRP,
- deren Aktivität durch Änderungen der Kultivierungstemperatur moduliert wird,
- deren Toxizität durch Mutagenese der Substrat-RNA in Kombination mit Selektion reduziert wird.

Im erfindungsgemäßen System ist die Substrat-RNA in einem bevorzugten Aspekt ein Primärtranskript, das von einer zellulären Polymerase oder von einer bakteriellen RNA-Polymerase wie T7, SP6 oder T3 *in vitro* oder in der Zelle synthetisiert wird. Die Promotoren auf dem Primärtranskript leiten sich bevorzugt aus einem Pflanzenvirus ab, von einem Vertreter der Familie *Tombusviridae,* insbesondere des Turnip-Crincle-Virus.
Das erfindungsgemäße Verfahren resultiert bevorzugt darauf, dass die Amplifikation einer RNA eintritt, die für ein Fremdgen kodiert, als Antisense-Transkript zu einem zellulären Transkript wirkt, als genomische RNA zur Herstellung rekombinanter Viren dient oder selbst enzymatische Aktivität aufweist.
Die Stärke natürlich vorkommender Promotoren für die RdRP wird in einem Aspekt der Erfindung durch Mutagenese verändert. Das Fremdgen und zumindest ein Promotor der RdRP in der Substrat-RNA liegen in einem bevorzugtem Aspekt der Erfindung in Antisense-Orientierung vor, wobei das Fremdgen ohne die RdRP nicht exprimiert werden kann, aber durch deren Expression aktiviert wird.
Die von der RdRP amplifizierte RNA trägt zur Verbesserung der Fremdgenexpression bevorzugt IRES-Elemente, Shunt-Donor und -Akzeptor und/oder Translationsenhancer. Sie enthält bevorzugt mindestens einen Polyadenylyltrakt und ist des weiteren bevorzugt dadurch charakterisiert, dass sie
- durch ein Ribozym prozessiert wird und/oder
- Signale zur Verpackung in eine Virushülle enthält und in Virushüllen verpackt wird.
Die Substrat-RNA enthält in weiteren Aspekten der Erfindung zwei gleiche oder unterschiedliche Promotoren in entgegengesetzter Orientierung, welche die Synthese von beiden RNA-Strängen initiieren und wodurch eine verstärkte Amplifikation induziert wird.
Die Expression der Substrat-RNA, der RdRP oder beider wird in einem Aspekt der Erfindung von einem auf zellulärer RNA-Polymerase II basierenden System reguliert.
Die RdRP wird in einem weiteren Aspekt der Erfindung ihrer enzymatischen Aktivität durch Änderungen in der Kultivierungstemperatur unterstützt, gesenkt oder abgeschaltet.
Das erfindungsgemäße Verfahren kann zur Auffindung von günstigen Mutationen in der RdRP oder im Fremdgen eingesetzt werden. Die günstigen Mutationen bewirken ihrerseits insbesondere eine verbesserte Replikation des Systems, eine verbesserte Leistung des Fremdgens und/oder eine geringere Toxizität des Fremdgens oder der RdRP.
Das erfindungsgemäße Verfahren ist in einem weiteren Aspekt so eingerichtet, dass es über zelluläre Ereignisse eingeschaltet oder aktiviert wird. Die Aktivierung indes
- bewirkt die Expression eines Fremdgens - einschließlich eines Reportergens, und/oder
- erfolgt über ein Fusionsprotein an der RdRP, und/oder
- dient zum Nachweis von Signaltransduktionswegen und der Translokation von intrazellulären Faktoren, und/oder
- erfolgt durch den Eintritt einer diffundierbaren Substanz oder eines Toxins oder über eine Infektion der Wirtszelle.
Bevorzugt für die Erfindung ist es, dass
- die Polymerase und die Promotoren aus einem Pflanzenvirus stammen
- die Polymerase von einem Vertreter der Familie *Tombusviridae* verwendet wird, insbesondere die Polymerase des Turnip-Crincle-Virus oder Carnation-Mottle-Virus
- als amplifizierte RNA eine modifizerte Satelliten-RNA des Turnip-Crincle-Virus oder eine modifizierte genomische RNA des Turnip-Crincle-Virus verwendet wird
- das Verfahren in Menschen-, Säugetier- oder Insektenzellen angewandt wird.

Das erfindungsgemäße Verfahren kann mit einem Testsystem (Testbesteck, Kit), bestehend aus zwei Komponenten durchgeführt werden: Komponente (1) ist eine Zelllinie, die im Genom das Gen für die RdRP stabil integriert trägt und dieses Gen konstitutiv, oder, unter Kontrolle eines regulierbaren Promotors, wie zum Beispiel das Tetrazyklin-System, nur nach Induktion exprimiert. Die bevorzugte Zelllinie lässt sich leicht transfizieren, so dass das Fremdgen mit den cis-aktiven Signalen für die RdRP einfach in diese Zellen einzubringen ist. Komponente (2) ist eine Sammlung von Expressionsplasmiden für die Substrat-RNA, wobei die bevorzugten Plasmide die cis-aktiven Signalsequenzen für die RdRP mit einer Region mit mehreren Schnittstellen für verschiedene Restriktionsenzyme kombinieren, so dass das Einfügen von Fremdgenen möglichst einfach ist. Die Expressionsplasmide verfügen über verschiedene Anordnungen der cis-aktiven Sequenzen, so dass je nach Wahl des Expressionsplasmids unterschiedlich starke Amplifikation oder Amplifikation gekoppelt mit Replikation möglich ist.
Außerdem verfügen die Expressionsplasmide über unterschiedlich starke Promotoren und Polyadenylierungssignale für die Expression des Primärtranskripts in der Zielzelle. Darüber hinaus verfügen die Expressionsplasmide über einen bakteriellen Promotor am Anfang und einer geeigneten Schnittstelle für ein Restriktionsenzym am Ende der Kassette für das Primärtranskript, so dass das Primärtranskript in vitro synthetisiert werden kann und als RNA, nicht als Plasmid-DNA, in die Zelle transfiziert werden kann. Zusätzlich zu der terminalen Schnittstelle für ein Restriktionsenzym trägt das Expressionsplasmid einen Terminator der Transkription für die bakterielle
Polymerase. Dadurch kann das Expressionsplasmid Primärtranskripte auch in der eukaryotischen Zelle über kotransfizierte bakterielle Polymerasen exprimieren. Zu diesem Zweck kann das Gen für die bakterielle Polymerase auch von einer eigenen Kassette auf dem Expressionsplasmid exprimiert werden, so dass eine Kotransfektion nicht notwendig ist.
Ein weiteres Testbesteck (Kit) zur Durchführung des erfindungsgemäßen Verfahrens besteht aus einer Zelllinie, die sowohl RdRP als auch die Expressionskassette für die Substrat-RNA stabil integriert im Genom trägt. Die RdRP ist hierbei jedoch unter Kontrolle eines induzierbaren Promotors, der auf eine Testsubstanz des Anwenders reagieren soll. Die Substrat-RNA trägt in replizierbarer und amplifizierbarer Kombination sowohl ein Gen für die RdRP als auch für ein Reporterprotein, also cis-aktive Signale für die RdRP auf beiden Seiten des Reportergens und des Gens für die RdRP. Dieses äußerst empfindliche System führt zu einer sehr schnellen und starken Expression des Reporterproteins, wenn die Zelle der Testsubstanz ausgesetzt wird. In einer Anwendung reagiert der induzierbare Promotor auf die Infektion durch ein anderes Virus, zum Beispiel Humanes Immunodefizienz Virus, und erlaubt so eine schnelle klinische Diagnostik. In einer weiteren Anwendung reagiert der induzierbare Promotor für die RdRP auf die Gegenwart von Schwermetallionen, und erlaubt so einen schnellen, quantifizierbaren Nachweis auf Umweltbelastungen ("Biosensor").

Die Erfindung soll anhand von Figuren und Ausführungsbeispielen näher erläutert werden, ohne auf diese beschränkt zu sein.

### AUSFÜHRUNGSBEISPIELE

Aus einem TCV-Virusinokulat in Form getrockneter, infizierter Blätter (DSMZ PV-0293; Deutsche Sammlung von Mikroorganismen und Zellkulturen in Braunschweig) wurde Gesamt-RNA über saures Phenol extrahiert (Trizol^{®}, Gibco BRL), in Gegenwart von Random Hexamer oder SatC-spezifischer Primer für 10 Minuten bei 80°C denaturiert und für 60 Minuten bei 42°C mit der Superscript II Reversen Transkriptase (Gibco BRL) in cDNA umgeschrieben. Als Vorlage für das Design von Primern für die PCR-Amplifikationen und nachfolgende Klonierungen dienten die GenBank-Sequenzen #M22445 für TCV und #X12750 für die Satelliten-RNA C (SatC).

### 1. KLONIERUNG DER RDRP

Das Gen für die RdRP (TCV 88-kD Protein) wurde mit den Primern i113 (ataccggtatgcctcttctacacac) und i114 (tagcggccgcttagagagttg) in einer PCR mit Taq (Qiagen GmbH, Max-Volmer-Straße 4, D-40724 Hilden) mit 10 Zyklen mit
94°C für 10 Sekunden Denaturierung,
56°C für 30 Sekunden Annealing und
68°C für 2 Minuten Polymerisierung, gefolgt von weitere 20 Zyklen mit
92°C für 10 Sekunden,
56°C für 30 Sekunden und
68°C für 2 Minuten, wobei mit jedem Zyklus diese Zeit um 20 Sekunden verlängert wurde,
aus dem cDNA-Gemisch amplifiziert. In den Primern befinden sich die Zielsequenzen für die Restriktionsenzyme Age I und Not I, so dass eine gerichtete Insertion in den Vektor pEGFP-N1 (Clontech Laboratories, Inc., 1020 East Meadow Circle, Palo Alto, CA 94303-4230, USA), unter Austausch gegen das Gen für GFP (green fluorescent protein), möglich sein sollte. Die Expression des 88-kD Proteins liegt dann unter Kontrolle des hCMV IE Promotors.

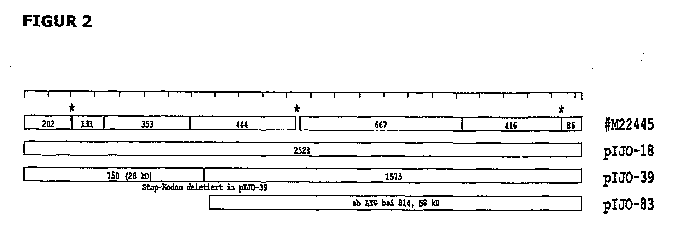

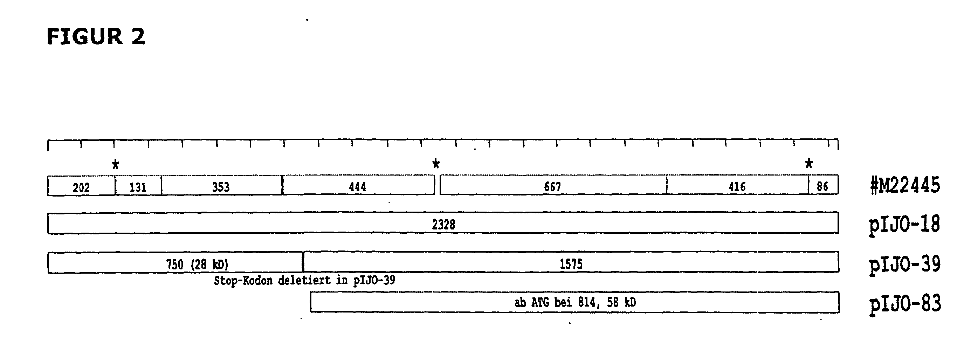

Im Gen für das 88-kD Protein waren die wichtigsten Abweichungen in drei unabhängigen Klonen von der Sequenz #M22445: (a) Von zwei veröffentlichten internen Stopkodons existiert das erste Stopkodon an Position 209 nicht. Anstelle von aga/gtc/aga/*TA/Ggt lautet die Sequenz dieser Experimente aga/ggt/cag/aTA/Ggt. (b) Im Gen wurde an der Position 1137 eine Insertion und an Position 1156 eine Deletion gefunden. Dadurch verschiebt sich der Leseraster gegenüber der publizierten Sequenz kurzfristig, und die Aminosäureabfolge lautet somit LDSLHDRPVSR anstelle von LDLYMTCRLSR. (c) Besonders überraschend ergab sich mit der Deletion bei Position 1156 von accggCt zu accggt eine Age I Schnittstelle. Diese Erscheinung war in allen PCR-Fragmenten zu finden und erschwerte die Klonierung erheblich, die anhand der veröffentlichten Sequenz geplant war. Die Klonierung gelang über einen Verdau mit Not I und einem Partialverdau, der die Age I Schnittstelle im Gen unberührt ließ; das derart
behandelte Amplifikationsprodukt wurde in die Age I Not I Stellen des Vektors pEGFP-N1 kloniert, womit der Leserahmen von EGFP durch die RdRP ersetzt wird und Plasmide pIJO-03, pIJO-18 und pIJO-19 entstanden. Diese drei Klone dienten der Gen-Sequenzbestimmung für das 88-kD Protein und sind Grundlage für die weiteren Arbeiten (zusammengefasst in Figur 2).
Laut Literatur [White et al., 1995 in Virology 211, 525-34] exprimieren die Tombusviridae die 88-kD RdRP über Suppression eines internen Amber-Stopkodons. Da nicht unbedingt anzunehmen ist, dass dieser Mechanismus der Suppression auch in Säugerzellen funktioniert, wurde das interne Stopkodon durch Site Directed Mutagenesis deletiert (gtccgcTAGgggtgc wird zu gtccgcgggtgc). Die Deletion lässt eine neue Schnittstelle für das Restriktionsenzym Sac II (ccgcgg) entstehen und erlaubt eine einfache Erfolgskontrolle durch Verdau; die resultierende Mutante wurde pIJO-39 genannt. Die verwendeten Mutageneseprimer waren i120 (gtcCGCGGGtgcttgcgg) und i121 (gcaagcaCCCGCGgacaa). In zwei getrennten PCR-Reaktionen wurden mit 250 ng pIJO-18 als Matritze in 50 µl Reaktionen Amplifikationsprodukte aus i116 (ATGGGCGGTAGGCGTGTA) und i121, sowie i117 (CAGGTTCAGGGGGAGGTG) und i120 gewonnen (beide Programme: 20 Zyklen mit jeweils 15 Sekunden 92°C, 30 Sekunden 56°C und 2 min 72°C; Pwo DNA-Polymerase (Roche Molecular Biochemicals, Sandhofer Straße 116, D-68305 Mannheim), 2 mM MgSO₄; die Primer i116 und i117 binden außerhalb der RdRP im Plasmid). Diese beiden Amplifikationsprodukte wurden über Agarosegelelektrophorese aufgereinigt und gemeinsam als Matrize in einer PCR-Reaktion mit den Primern i116 und i117 mit dem gleichen Thermocycler-Programm wie für die beiden Halbreaktionen eingesetzt. Der Erfolg des Einbaus der mutagenisierten Sequenz (die in den komplementären Primern i120 und i121 enthalten war) wurde über Restriktionsverdau mit Sac II und Sequenzierung überprüft und bestätigt (SEQUENZ #1/SEQ ID NO:1, siehe auch Figur 2).

### 2. KLONIERUNG DER SUBSTRAT-RNA

SatC wurde mit Primer i109 (GGGCAGGCCC) aus der oben beschriebenen Gesamt-RNA mit SuperScript II Reverse Transkriptase in cDNA umgesetzt und mit den Primern i110 (cccgggcaggccccc) und i112 (cccgggataactaagggtttcatac) aus dem cDNA-Gemisch wie für die RdRP beschrieben amplifiziert. Mittels der unkodierten Adenosyl-Überhänge in Amplifikationsprodukten der Taq-Polymerase wurde dieses Produkt in den Vektor pGEM-T (Invitrogen) subkloniert. Jeweils drei unabhängig voneinander gewonnene Klone (Plasmide pIJO-04, pIJO-20 und pIJO-21) von SatC wurden sequenziert. Hierbei ergaben sich erhebliche Abweichungen zu der veröffentlichten Sequenz. Da diese Änderungen jedoch den jeweiligen drei Klonen gemeinsam waren, wurden sie in die endgültige Sequenz dieser Arbeit aufgenommen (SEQUENZEN #2 und #3/SEQ ID NOs:3 and 4 die Termini mit Schnittstellen für Sma I versehen {cccggg}; SEQUENZ #4/SEQ ID NO:5 für pIJO-20).
In SatC waren die wichtigsten Abweichungen der drei unabhängigen Klone von der Sequenz #X12750: (1) Im viralen Promotor für den genomischen Strang wurde zwei Nukleotidaustausche und eine Insertion gefunden. Dadurch ändert sich die Sequenz von ccGTccga zu ccCCcGcga (2) Im zentralen Bereich zeigten sich unerwartet AT-reiche Insertionen von insgesamt 76 Basen.
Anhand der erhaltenen Sequenzen, und unter Berücksichtigung früherer Arbeiten [Carpenter & Simon 1998 in Nucl. Acids Res. 26, 2426-32 und Simon et al. 1988 in EMBO J 7, 2645-51] wurden für diese Arbeit folgende virale Promotoren ermittelt (Sequenzen des sense-Strangs):
... tatgaaacccttagttatccc-OH, und
... agcctccctcctcgcgggggggggcctgccc-OH
Folgende Plasmide wurden für die Expression des Substrat-Transkripts der RdRP erstellt:
pIJO-60 und pIJO-61: Ein 1334 bp langes EcoR I EcoR I Fragment mit EMCV IRES gefolgt von GFP aus pIJO-17 wurde in die BstE II Schnittstelle in der SatC Region von pIJO-20 gesetzt; die Termini von Insert und Vektor wurden vor der Ligation mit Klenow-Polymerase aufgefüllt. Durch diese Klonierung entstanden pIJO-27 (IRES/GFP in gleicher Orientierung wie SatC) und pIJO-28 (IRES/GFP entgegengesetzt orientiert zu SatC). Diese beiden SatC-Kasetten mit dem IRES/GFP-Insert wurden mit Sma I als 1735 bp-Fragmente isoliert und in die Sma I Schnittstelle von pIJO-24 inseriert. pIJO-24 ist ein Vektor, in dem der Sma I Schnittstelle auf der 5'-Seite ein CMV (Cytomegalovirus) und T7-Promotor und auf der 3'-Seite das Ribozym des Hepatitis-Delta-Virus, der T7-Terminator und das HSV Thymidine Kinase-Polyadenylierungssignal folgen. Nach der Prozessierung durch das Ribozym sollten der TCV-RdRP exakte (wildtypische) Termini im Primärtranskript zur Verfügung stehen. Durch die Klonierung enstehen insgesamt vier mögliche Kombination, wovon zwei für diese Experimente interessant sind: pIJO-60 exprimiert SatC in sense und IRES/GFP in anti-sense Orientierung, pIJO-61 exprimiert sowohl SatC als auch IRES/GFP in anti-sense-Orientierung. Durch die anti-sense-Orientierung der IRES/GFP-Kasette wird gewährleistet, dass die Zelle nur nach erfolgreicher Transkription vom Primärtranskript durch die RdRP den GFP-Reporter exprimieren kann.
Die IRES vor GFP soll vor allem im Konstrukt pIJO-60 die Expression von GFP durch Umgehung von drei natürlichen ATG-Kodons vor dem Gen für den Reporter ermöglichen (siehe SEQUENZ #2/SEQ ID NO:3). Da jedoch der Einfluss der Sekundärstruktur einer IRES auf die Prozessivität durch die RdRP nicht vorhergesagt werden kann, sind weitere Konstrukte für die Bereitstellung von Substrat-RNA ohne IRES erstellt worden. Auch für die beiden Plasmide pIJO-79 und pIJO-80 liegt im Primärtranskript GFP in anti-sense vor. Als Ausgangspunkt wurde diesmal nur die Expression von SatC in anti-sense gewählt, in der Hoffnung, dass wie bei anderen RNA-Viren auch bei TCV eine assymetrische Replikation der beiden Stränge, also eine relative Anreicherung des Strangs in genomischer Orientierung erfolgt. Die Ursache für diese Beobachtung liegt darin, dass der Promotor auf dem anti-sense-Strang der stärkere ist, und damit ein besserer Nachweis der Reporterexpression vom Primärtranskript dieser Orientierung möglich sein sollte. Ein weiterer Vorteil des anti-sense-Strangs ist der kurze Leader frei von AUG-Kodons vor der BstE II Schnittstelle; im Plusstrang würden Startkodons vor GFP liegen, so dass bei der hier beschriebenen Klonierung auf eine IRES nicht verzichtet werden sollte.
Für die Herstellung von pIJO-79 (SEQUENZ #5/SEQ ID NO:6) und pIJO-80 wurde GFP als Klenow-behandeltes Nco I/Xba I Fragment aus pEGFP (Clontech) in mit BsteE II und Klenow vorbereiteten pIJO-20 gesetzt, womit pIJO-78 entsteht. Die GFP/SatC-Kasette wurde als 1139 bp Sma I-Fragment zwischen (im Vergleich zu CMV schwächeren) hPGK-Promotor und CMV polyA-Signal gesetzt, womit pIJO-80 entsteht. Für das Plasmid pIJO-79 wurde das Sma I-Fragment zwischen T7-Promotor und HDV-Ribozym/T7-Terminator gesetzt.
Die Expression des Primärtranskripts über eine Kotransfektion mit T7-RNA-Polymerase erlaubt die Expression vor allem, wenn ein potentielles polyA-Signal (AATAAA) im anti-sense-Strang von SatC (siehe SEQUENZ #3/SEQ ID NO:4) für die Polymerase II aktiv sein sollte.

### 3. MODIFIKATION DER RdRP

Bei den Sequenzuntersuchungen fanden wir 60 bp downstream dem hier mutagenisierten TAG-Stopkodon überraschend ein internes ATG (an Position 811 in SEQUENZ #1/SEQ ID NO:1) in sehr guter Kozak-Umgebung [Kozak 1989 in J Cell Biol 108, 229-41].
Kozak: gccgccgccAUGg (oder gccgccaccAUGg)
TCV RdRP: aacccggccAUGc
Obwohl in der Literatur die Expression der RdRP als 88-kD Protein in einer Fusion mit P28 beschrieben wird [White et al., 1995 in Virology 211, 525-34], halten wir es mit den bislang bekannten Daten für vereinbar, dass die RdRP auch als etwa 58-kD Protein nach Re-Initierung der Translation nach dem Stop für P28 exprimiert werden kann, und möglicherweise gerade dieses Genprodukt die aktive RdRP darstellt. Wir haben daher ein weiteres Expressionsplasmid für die TCV-RdRP erstellt, pIJO-83, welches nur das carboxyl-terminale Fragment von 1515 bp (505 Aminosäuren) mit einem theoretischen Molekulargewicht von 58 kD exprimiert. Für die Herstellung von pIJO-83 wurde ein Sac II Not I Fragment aus pIJO-39 (der TAG-deletierten Mutante) in die gleichen Schnittstellen des Vektors pEGFP-N1 im Austausch gegen GFP transferiert. Die Schnittstelle für Sac II war durch die Deletion des internen Stop-Kodons bei der Klonierung von pIJO-39 aus pIJO-18 entstanden.

### 4. TRANSFEKTION DER EXPRESSIONSPLASMIDE

Plasmid pIJO-39 (TCV 88-kD Protein) wurde mit SatC-Expressionsplasmiden pIJO-60, pIJO-61, pIJO-79 oder pIJO-80 in 5 x 10^5 293 Zellen mit Polyfect (Qiagen) kotransfiziert; bei Transfektionen von SatC-Plasmiden mit T7-Promotor wurde außerdem ein Expressionsplasmid für die T7-RNA-Polymerase unter Kontrolle des CMV-Promotors mit in das Transfektionsgemisch gegeben (also für pIJO-60, pIJO-61 und pIJO-79).
In anderen Experimenten wurde das Plasmid pIJO-83 (TCV 58-kD Protein) wie für pIJO-39 beschrieben mit SatC-Expressionsplasmiden in 293 Zellen kotransfiziert.
In wieder anderen Experimenten wurden Kombinationen aus pIJO-83 und pIJO-18 (die wildtypische RdRP-Sequenz mit Stopkodon nach dem 28-kD Gen) zusammen mit SatC-Expressionsplasmiden in 293 Zellen kotransfiziert.
Als Negativkontrolle dienten Transfektionen, in denen das Expressionsplasmid für die RdRP durch ein Plasmid ersetzt wurden, das in Säugerzellen keine Proteine exprimiert (pUC-19 oder pBluescript).
Expression des Reporterproteins wurde über Fluoreszenzmikroskopie mit einer Anregung bei 470-490 nm und einem Sperrfilter von 515 nm beobachtet.

### LEGENDE ZU DEN FIGUREN

### FIGUR 1

zeigt einen schematischen Vergleich von Amplifikation des Primärtranskripts, gekoppelter Amplifikation/Replikation, und Amplifikation gekoppelt mit assymetrischer Replikation. Das Primärtranskript (a) mit Fremdgen in anti-sense (grauer Pfeil) und viralen Promotor (VP) wird durch die RdRP (Kreissymbol) erkannt und in Sekundärtranskripte umgesetzt; Promotoren, die in der falschen Orientierung vorliegen und daher nicht erkannt werden können, sind durch die rotierte Buchstabenfolge VP symbolisiert (b). Trägt das Primärtranskript am 5' Ende einen viralen Promotor, dann kann das Sekundärtranskript als Substrat für weitere anti-sense Transkripte (c) dienen, die wiederum sense-RNA hervorbringen. Für eine asymmetrische Replikation besitzt das Primärtranskript zwei interne VPs, die ein Gen der bicistronischen Primär-RNA einrahmen. Das zweite Gen (hier gestrichelt dagestellt) wird von einem Transkript exprimiert, das im internen Promotor im Primärtranskript initiiert. Es kann in diesem Beispiel nicht repliziert werden, da am 5'-Terminus des Primärtranskripts ein VP weggelassen wurde. Das kleine Transkript jedoch, das aus dem Sekundärtranskript durch interne Initiierung hervorgeht, trägt an beiden Termini VPs und kann somit repliziert/amplifiziert werden (d).
Die RdRP selbst ist in diesen Beispiel nicht derart auf dem Primärtranskript kodiert, dass sie von viralen Promotoren auf beiden Seiten eingeschlossen wird. Das System kommt somit, im Gegensatz zu herkömmlicher viraler Replikation, mit dem Abschalten der RdRP-Expression zum Erliegen. Außerdem kann in den Beispielen der FIG. 1 das Fremdgen nur von Sekundärtranskripten exprimiert werden, da es auf dem Primärtranskript in nicht-kodierender Orientierung vorliegt.

### FIGUR 2

zeigt eine schematische Darstellung der klonierten Gene für die TCV-RdRP im Vergleich zur publizierten Sequenz #M22445 in der GenBank. Unterschiede zwischen #M22445 und der korrigierten Sequenz dieser Arbeit sind durch die vertikalen Striche in den Balken angedeutet; mit einem Stern (*) gekennzeichnete Unterschiede führen zu Änderungen in der Aminosäuresequenz. Zusammenfassend: pIJO-18 trägt das wildtypische TCV-RdRP-Gen, pIJO-39 eine gezielte Deletion eines internen Stop-Kodons, und pIJO-83 eine aminoterminale Deletion des 88-kD Proteins. Translation des 58-kD Proteins von pIJO-83 beginnt mit einem internen ATG nach dem Stopkodon für das 28-kD Protein.

### ABKÜRZUNGSVERZEICHNIS

- Age I: Restriktionsenzym
- ATG: Initiatorkodon der Translation
- AUG: Initiatorkodon der Translation, RNA-Sequenz
- BstE II: Restriktionsenzym
- Cap-: Methyl-GpppG-5'-terminale Modifikation an mRNA
- CMV: Cytomegalovirus
- cDNA: complementary DNA, copy DNA
- DNA: desoxyribonucleic acid (Desoxyribonukleinsäure)
- DSMZ: Deutsche Sammlung von Mikroorganismen und Zellkulturen Braunschweig
- EGFP: enhanced green fluorescent protein, Reporterprotein
- GFP: green fluorescent protein
- hCMV IE: Cytomegalovirus des Menschen, Immediate Early Promotor
- HDV: Hepatitis-Delta-Virus
- hPGK: Gen der humanen Phosphoglyceratkinase
- HSV: Herpes-Simplex-Virus
- IkB: Inhibitor-kappa-B
- IRES: internal ribosomal entry site
- kD: kilo-Dalton, 1000 g/mol
- mRNA: Messenger-Ribonucleinsäure, Boten-RNA
- P88: 88kD-Genprodukt, mögliche RdRP von TCV
- PCR: polymerase chain reaction (Polymerase-Kettenreaktion)
- PGK: Phosphoglyceratkinase
- Pwo: Pyrococcus woesei
- pUC: Plasmid pUC
- RdRP(s): RNA-abhängige RNA-Polymerase
- RNA: Ribonucleinsäure (ribonucleic acid)
- Sac II: Restriktionsenzym
- Sat: Satelliten
- SatC: Satelliten-RNA C
- Sma: Restriktionsenzym
- ssRNA: single stranded RNA, einzelsträngige RNA
- TAG: Sequenz auf DNA für Stopkodon
- TCV: Turnip-Crinkle-Virus (Turnip Crinkle Virus)
- tRNA: Transport-RNA
- VP(s): virale Promotoren

## Patentansprüche

1. Verfahren zur Amplifikation von Nukleinsäuren in tierischen Zellen *in vitro,* umfassend das Einbringen
(i) einer RNA-abhängigen RNA-Polymerase (RdRP) eines Pflanzenvirus und
(ii) einer RNA, die Promotoren oder cis-aktive Signale enthält, welche von der RdRP erkannt werden (Substrat-RNA)
in tierische Zellen mit Ausnahme humaner embryonaler Stammzellen.

2. Verfahren nach Anspruch 1, wobei
(a) die tierischen Zellen ausgewählt sind aus Säugerzellen einschließlich Humanzellen, Insekten-, Wurm- und Amphibienzellen; und/oder
(b) die amplifizierte Nukleinsäure eine RNA ist; und/oder
(c) die Substrat-RNA (i) ein Primärtranskript ist, welches durch eine zellulär vorliegende RNA-Polymerase der tierischen Zelle, insbesondere der RNA-Polymerase II, oder eine rekombinant eingebrachten Polymerase in der tierischen Zelle synthetisiert wurde, oder (ii) *in vitro* synthetisiert und von außen in die tierische Zelle transfiziert wurde; und/oder
(d) die RdRP durch ein RdRP-Gen, welches in die tierische Zelle eingebracht wird, codiert wird; und/oder
(e) die amplifizierte RNA (i) eine kodierende RNA ist, oder (ii) auch ohne Translation eine Wirkung entfaltet.

3. Verfahren nach Anspruch 1 oder 2, wobei
(a) die RdRP eines Vertreters der Tombusviridae, Bromoviridae oder Potyviridae, bevorzugt der Tombusviridae, besonders bevorzugt des Turnip-Crincle-Virus verwendet wird; und/oder
(b) die RdRP durch die Substrat-RNA oder durch ein gesondertes Transkript getrennt von ihrem Promoter codiert wird; und/oder
(c) die in der Substrat-RNA enthaltenen Promotoren pflanzenviral sind; und/oder
(d) die Expression des Primärtranskripts, der RdRP oder beider von einem auf zellulärer RNA-Polymerase II basierenden System reguliert wird.

4. Verfahren nach einem oder mehreren der Ansprüche 1 bis 3, wobei
(a) die RdRP, die normalerweise in Pflanzenzellen aktiv ist und deren Gen aus Pflanzenzellen gewonnen werden kann, durch die Sequenz #1 (SEQ ID NO:1) codiert wird; und/oder
(b) die Aktivität der RdRP durch Änderungen in der Kultivierungstemperatur moduliert wird; und/oder
(c) die Stärke natürlich vorkommender Promotoren für die RdRP durch Mutagenese verändert ist; und/oder
(d) die Toxizität der RdRP durch Mutagenese der Substrat-RNA in Kombination mit Selektion reduziert ist; und/oder
(e) als Substrat-RNA entweder eine modifizerte Satelliten-RNA, bevorzugt eine modifizerte Satelliten-RNA des Turnip-Crincle-Virus, oder eine modifizierte genomische RNA des Turnip-Crincle-Virus verwendet wird.

5. Verfahren nach einem oder mehreren der Ansprüche 1 bis 4, wobei die Substrat-RNA und das Transkript, welches die RdRP codiert, unabhängige voneinander exprimiert werden, und die Substrat-RNA ein Fremdgen und darauf folgend einen viralen Promoter in nicht codierender Orientierung enthält.

6. Verfahren nach einem oder mehreren der Ansprüche 1 bis 4, wobei die Substrat-RNA ein Fremdgen enthält, das von zwei viralen Promotoren flankiert wird.

7. Verfahren nach Anspruch 6, wobei
(a) die Substrat-RNA und das Transkript, welches die RdRP codiert, unabhängig voneinander sind, und das RdRP-Gen von zwei viralen Promotoren flankiert wird; oder
(b) die Substrat-RNA für RdRP und Fremdgen codiert und an beiden Termini funktionelle virale Promotoren enthält.

8. Verfahren nach einem oder mehreren der Ansprüche 1 bis 7, wobei die Amplifikation einer RNA eintritt, die für ein Fremdgen kodiert, als Antisense-Transkript zu einem zellulären Transkript wirkt, als genomische RNA zur Herstellung rekombinanter Viren dient oder selbst enzymatische Aktivität hat.

9. Verfahren nach einem oder mehreren der Ansprüche 1 bis 8, wobei
(a) die Substrat-RNA IRES-Elemente, Shunt-Donor und -Akzeptor oder Translationsenhancer zur Verbesserung der Fremdgenexpression trägt; und/oder
(b) die Substrat-RNA zwei gleiche oder unterschiedliche Promotoren in entgengesetzter Orientierung enthält, welche die Synthese von beiden RNA-Strängen initiieren, wodurch eine verstärkte Amplifikation induziert wird; und/oder
(c) die von der RdRP amplifizierte RNA mindestens einen Polyadenylyltrakt enthält; und/oder
(d) die von der RdRP amplifizierte RNA durch ein Ribozym prozessiert wird; und/oder
(e) die von der RdRP amplifizierte RNA Signale zur Verpackung in eine Virushülle enthält und in Virushüllen verpackt wird.

10. Verfahren nach einem oder mehreren der Ansprüche 1 bis 9, wobei das Verfahren
(a) zur Auffindung von günstigen Mutationen in der RdRP oder im Fremdgen eingesetzt wird, wobei die günstigen Mutationen insbesondere zu einer verbesserten Replikation des Systems, einer verbesserten Leistung des Fremdgens oder einer geringeren Toxizität des Fremdgens führen; und/oder
(b) über zelluläre Ereignisse eingeschaltet oder aktiviert wird, wobei insbesondere die Aktivierung die Expression eines Fremdgens - einschließlich eines Reportergens - bewirkt, die Aktivierung über ein Fusionsprotein an der RdRP erfolgt, die Aktivierung zum Nachweis von Signaltransduktionswegen oder der Translokation von intrazellulären Faktoren dient, die Aktivierung durch den Eintritt einer diffundierbaren Substanz oder eines Toxins erfolgt oder die Aktivierung über eine Infektion der Wirtszelle erfolgt.

11. Verfahren nach einem oder mehreren der Ansprüche 1 bis 10, das geeignet ist
(a) zur Amplifikation von Nukleinsäuren, insbesondere zur Amplifikation von RNA in tierischen Zellen; und/oder
(b) zur Auffindung von günstigen Mutationen in der RdRP oder im Fremdgen, wobei die günstigen Mutationen insbesondere zu einer verbesserten Replikation des Systems, einer verbesserten Leistung des Fremdgens oder einer geringeren Toxizität des Fremdgens führen; und/oder
(c) zur Expression eines Fremdgens einschließlich eines Reportergens, zum Nachweis von Signaltransduktionswegen oder der Translokation von intrazellulären Faktoren; und/oder
(d) zur Kontrolle der Genexpression.

12. Tierische Zelle mit Ausnahme humaner embryonaler Stammzellen, enthaltend
(i) eine RNA-abhängigen RNA-Polymerase (RdRP) eines Pflanzenvirus und
(ii) eine RNA, die Promotoren oder cis-aktive Signale enthält, welche von der RdRP erkannt werden (Substrat-RNA),
wie in einem oder mehreren der Ansprüche 1 bis 11 definiert.

13. Isolierte Transkripte, die zum Einbringen
(i) einer RNA-abhängigen RNA-Polymerase (RdRP) eines Pflanzenvirus und
(ii) einer RNA, die Promotoren oder cis-aktive Signale enthält, welche von der RdRP erkannt werden (Substrat-RNA), wie in einem oder mehreren der Ansprüche 1 bis 11 definiert, in tierische Zellen mit Ausnahme humaner embryonaler Stammzellen geeignet sind und
(a) welche eine RdRP codieren; und
(b) welche ein Fremdgen und zumindest einen Promoter, der durch RdRP erkannt wird, enthalten, wobei beide in Antisense-Orientierung vorliegen und das Fremdgen ohne die RdRP nicht exprimiert werden kann, aber durch deren Expression aktiviert wird.

14. Isolierte Transkripte nach Anspruch 13, wobei RdRP und Promotoren aus einem Pflanzenvirus stammen, wobei bevorzugt die Polymerase von einem Vertreter der Familie Tombusviridae, insbesondere dem Turnip-Crincle-Virus stammt.

15. Isolierte Transkripte nach Anspruch 13 oder 14,
(a) die Promotoren enthalten, deren Stärke gegenüber den natürlich vorkommenden Promotoren durch Mutagenese verändert wurde; und/oder
(b) deren amplifizierte RNA IRES-Elemente, Shunt-Donor und -Akzeptor oder Translationsenhancer zur Verbesserung der Fremdgenexpression tragen; und/oder
(c) deren amplifizierte RNA vorzugsweise mindestens einen Polyadenylyltrakt enthält, durch ein Ribozym prozessiert wird, Signale zur Verpackung in eine Virushülle enthält und in Virushüllen verpackt wird, für ein Gen oder mehrere Gene kodiert, für Gene in unterschiedlichen Orientierungen kodiert, für eine RNA steht, die ohne Translation eine Wirkung in der Zelle entfaltet, wie Ribozyme, ribosomale RNA oder Antisense-Konstrukte und/oder für eine genomische oder subgenomische RNA eines anderen Virus kodiert.

16. Verwendung der Transkripte nach einem oder mehreren der Ansprüche 13 bis 15 zur in-vitro-Amplifikation von Nukleinsäuren, insbesondere zur Amplifikation von RNA in tierischen Zellen mit Ausnahme humaner embryonaler Stammzellen.

17. Medikament zur Gentherapie, Vakzinierung oder therapeutischen Vakzinierung enthaltend ein Transkript nach einem oder mehreren der Ansprüche 13 bis 15.

## Claims

1. A method for the in vitro amplification of nucleic acids in animal cells, comprising the introduction of
(i) an RNA-dependent RNA polymerase (RdRP) of a plant virus; and
(ii) an RNA which contains promoters or cis-active signals recognized by said RdRP (substrate RNA);
into animal cells, except for human embryonic stem cells.

2. The method according to claim 1, wherein:
(a) said animal cells are selected from mammal cells including human cells, insect, worm and amphibian cells; and/or
(b) said amplified nucleic acid is an RNA; and/or
(c) said substrate RNA (i) is a primary transcript synthesized within said animal cell by a cellular RNA polymerase of the animal cell, especially RNA polymerase II, or a recombinantly introduced polymerase, or (ii) has been synthesized in vitro and transfected from outside into said animal cell; and/or
(d) said RdRP is encoded by an RdRP gene introduced into the animal cell; and/or
(e) said amplified RNA (i) is a coding RNA, or (ii) displays its action even without translation.

3. The method according to claim 1 or 2, wherein:
(a) the RdRP of a member of the Tombusviridae, Bromoviridae or Potyviridae family, preferably Tombusviridae, more preferably the turnip crinkle virus, is used; and/or
(b) said RdRP is encoded by said substrate RNA or by a separate transcript separately from its promoter; and/or
(c) the promoters contained in said substrate RNA are derived from a plant virus; and/or
(d) the expression of the primary transcript, of the RdRP or both is regulated by a system based on cellular RNA polymerase II.

4. The method according to one or more of claims 1 to 3, wherein:
(a) said RdRP, which is normally active in plant cells and whose gene can be obtained from plant cells, is encoded by the sequence #1 (SEQ ID No. 1); and/or
(b) the activity of said RdRP is- modulated by changes in the culturing temperature; and/or
(c) the strength of naturally occurring promoters for the RdRP is modified by mutagenesis; and/or
(d) the toxicity of the RdRP is reduced by mutagenesis of the substrate RNA in combination with selection; and/or
(e) either a modified satellite RNA, preferably a modified satellite RNA of turnip crinkle virus, or a modified genomic RNA of turnip crinkle virus is used as said substrate RNA.

5. The method according to one or more of claims 1 to 4, wherein the substrate RNA and the transcript which encodes the RdRP are expressed independently of each other, and the substrate RNA contains a foreign gene and subsequently a viral promoter in non-coding orientation.

6. The method according to one or more of claims 1 to 4, wherein the substrate RNA contains a foreign gene flanked by two viral promoters.

7. The method according to claim 6, wherein:
(a) the substrate RNA and the transcript coding the RdRP are independent of each other, and the RdRP gene is flanked by two viral promoters; or
(b) the substrate RNA codes for RdRP and a foreign gene and contains functional viral promoters at both termini.

8. The method according to one or more of claims 1 to 7, wherein the amplification of an RNA occurs which codes for a foreign gene, acts as an antisense transcript of a cellular transcript, serves as a genomic RNA for the preparation of recombinant viruses, or has itself enzymatic activity.

9. The method according to one or more of claims 1 to 8, wherein:
(a) the substrate RNA contains IRES elements, shunt donor and acceptor, or translation enhancers for improving foreign gene expression; and/or
(b) the substrate RNA contains two similar or dissimilar promoters in opposite orientation which initiate the synthesis from both RNA strands to induce an enhanced amplification; and/or
(c) the RNA amplified from the RdRP contains at least one polyadenylation tract; and/or
(d) the RNA amplified from the RdRP is processed by a ribozyme; and/or
(e) the RNA amplified from the RdRP contains signals for packaging into a viral envelope and is packaged into viral envelopes.

10. The method according to one or more of claims 1 to 9, wherein said method:
(a) is employed for finding favorable mutations in the RdRP or in the foreign gene, wherein said favorable mutations cause, in particular, an improved replication of the system, an improved performance of the foreign gene, or a lower toxicity of the foreign gene; and/or
(b) is switched on or activated by cellular events, in particular wherein the activation causes expression of a foreign gene, including a reporter gene, the activation is effected through a fusion protein at the RdRP, the activation serves for the detection of signal transduction pathways or the translocation of intracellular factors, the activation is effected by the entering of a diffusible substance or a toxin, or the activation is effected through infection of the host cell.

11. The method according to one or more of claims 1 to 10, which is suitable for:
(a) the amplification of nucleic acids, especially the amplification of RNA, in animal cells; and/or
(b) finding favorable mutations in the RdRP or in the foreign gene, wherein said favorable mutations cause, in particular, an improved replication of the system, an improved performance of the foreign gene, or a lower toxicity of the foreign gene; and/or
(c) expression of a foreign gene, including a reporter gene, for the detection of signal transduction pathways or the translocation of intracellular factors; and/or
(d) controlling gene expression.

12. An animal cell, except for human embryonic stem cells, containing:
(i) an RNA-dependent RNA polymerase (RdRP) of a plant virus; and
(ii) an RNA which contains promoters or cis-active signals recognized by said RdRP (substrate RNA);
as defined in one or more of claims 1 to 11.

13. Isolated transcripts suitable for introducing:
(i) an RNA-dependent RNA polymerase (RdRP) of a plant virus and
(ii) an RNA which contains promoters or cis-active signals recognized by said RdRP (substrate RNA);
as defined in one or more of claims 1 to 11 into animal cells, except for human embryonic stem cells; and
(a) which encode an RdRP; and
(b) which contain a foreign gene and at least one promoter recognized by RdRP, both being in antisense orientation, and the foreign gene cannot be expressed without said RdRP, but is activated by its expression.

14. The isolated transcripts according claim 13, wherein the RdRP and promoters are derived from a plant virus, wherein the polymerase is preferably derived from a member of the Tombusviridae family, especially turnip crinkle virus.

15. The isolated transcripts according claim 13 or 14:
(a) which contain promoters the strength of which as compared to naturally occurring promoters has been changed by mutagenesis; and/or
(b) whose amplified RNA bears IRES elements, shunt donor and acceptor, or translation enhancers for improving foreign gene expression; and/or
(c) whose amplified RNA preferably contains at least one polyadenylation tract, is processed by a ribozyme, contains signals for packaging into a viral envelope and is packaged into viral envelopes, codes for a gene or several genes, codes for genes in different orientations, represents an RNA which displays its action in cells without translation, such as ribozymes, ribosomal RNA or antisense constructs, and/or codes for a genomic or subgenomic RNA of another virus.

16. Use of the transcripts according to one or more of claims 13 to 15 for the in vitro amplification of nucleic acids, especially for the amplification of RNA in animal cells, except for human embryonic stem cells.

17. A medicament for gene therapy, vaccination or therapeutic vaccination, containing a transcript according to one or more of claims 13 to 15.

## Revendications

1. Procédé *in vitro* d'amplification d'acides nucléiques dans des cellules animales, comprenant l'introduction
(a) d'une ARN-polymérase fonction de l'ARN (RdRP) d'un virus de plante et
(b) d'un ARN qui contient des promoteurs ou des signaux cis-actifs qui sont reconnus par la RdRP (ARN substrat)
dans des cellules animales à l'exception de cellules souches embryonnaires humaines.

2. Procédé selon la revendication 1, dans lequel
(a) les cellules animales sont choisies parmi des cellules de mammifères, y compris des cellules humaines, des cellules d'insectes, de vers et d'amphibiens ; et/ou
(b) l'acide nucléique amplifié est un ARN ; et/ou
(c) l'ARN substrat (i) est un transcrit primaire, lequel a été synthétisé dans la cellule animale par une ARN-polymérase de la cellule animale existant dans la cellule, en particulier de l'ARN-polymérase II, ou par une polymérase introduite de façon recombinante, ou (ii) a été synthétisé in vitro et a été transfecté de l'extérieur dans la cellule animale ; et/ou
(d) la RdRP est codée par un gène de RdRP qui est introduit dans la cellule ; et/ou
(e) l'ARN amplifié (i) est un ARN codé ou (ii) développe son effet même sans traduction.

3. Procédé selon la revendication 1 ou 2, dans lequel
(a) on utilise la RdRP d'un représentant des Tombusviridae, des Bromoviridae ou des Potyviridae, de préférence des Tombusviridae, de façon particulièrement préférée du virus Turnip-Crincle ; et/ou
(b) la RdRP est codée par l'ARN substrat ou par un transcrit spécial, séparément de son promoteur ; et/ou
(c) les promoteurs contenus dans l'ARN substrat sont de la nature de virus de plante ; et/ou
(d) l'expression du transcrit primaire, de la RdRP ou des deux est réglée par un système se basant sur l'ARN-polymérase II cellulaire.

4. Procédé selon l'une ou plusieurs des revendications 1 à 3, dans lequel
(a) la RdRP qui est normalement active dans les cellules de plantes et dont le gène peut être obtenu à partir de cellules de plantes est codée par la SEQUENCE #1 (SEQ ID NO : 1) ; et/ou
(b) l'activité de la RdRP est modulée par des modifications dans la température de culture ; et/ou
(c) la force de promoteurs pour la RdRP existant dans la nature est modifiée par mutagenèse ; et/ou
(d) la toxicité de la RdRP est réduite par mutagenèse de l'ARN substrat en combinaison avec une sélection ; et/ou
(e) comme ARN substrat est utilisé soit un ARN satellite, de préférence un ARN satellite du virus Turnip-Crincle, soit un ARN génomique modifié du virus Turnip-Crincle.

5. Procédé selon l'une ou plusieurs des revendications 1 à 4, dans lequel l'ARN substrat et le transcrit qui code la RdRP sont exprimés indépendamment l'un de l'autre, et dans lequel l'ARN substrat contient un gène étranger et un promoteur viral qui suit ce dernier dans une orientation non codante.

6. Procédé selon l'une ou plusieurs des revendications 1 à 4, dans lequel l'ARN substrat contient un gène étranger qui est flanqué de deux promoteurs viraux.

7. Procédé selon la revendication 6, dans lequel
(a) l'ARN substrat et le transcrit qui code la RdRP sont indépendants l'un de l'autre et le gène RdRP est flanqué de deux promoteurs viraux ; ou
(b) l'ARN substrat code pour la RdRP et pour le gène étranger et contient des promoteurs viraux fonctionnels sur les deux terminaisons.

8. Procédé selon l'une ou plusieurs des revendications 1 à 7, dans lequel se produit l'amplification d'un ARN qui code pour un gène étranger, agit comme transcrit anti-sens d'un transcrit cellulaire, sert d'ARN génomique pour la fabrication de virus recombinants ou a lui-même une activité enzymatique.

9. Procédé selon l'une ou plusieurs des revendications 1 à 8, dans lequel
(a) l'ARN. substrat porte des éléments IRES, un donneur ou un accepteur de shunt ou un amplificateur de traduction pour l'amélioration de l'expression du gène étranger ;
(b) l'ARN substrat contient deux promoteurs identiques ou différents dans des orientations opposées, lesquels initialisent la synthèse des deux brins d'ARN, par quoi une amplification renforcée est induite ; et/ou
(c) l'ARN amplifié par la RdRP contient au moins un tractus polyadénylyle ; et/ou
(d) l'ARN amplifié par la RdRP est procédé par un ribozyme ; et/ou
(e) l'ARN amplifié par la RdRP contient des signaux pour l'emballage dans une enveloppe de virus et est emballé dans des enveloppes de virus.

10. Procédé selon l'une ou plusieurs des revendications 1 à 9, sachant que le procédé
(a) est utilisé pour la découverte de mutations avantageuses dans la RdRP ou dans le gène étranger, sachant que les mutations avantageuses conduisent en particulier à une meilleure réplication du système, à un rendement amélioré du gène étranger ou à une toxicité moindre du gène étranger; et/ou
(b) est lancé ou activé par l'intermédiaire d'événements cellulaires, sachant qu'en particulier l'activation produit l'expression d'un gène étranger - y compris d'un gène révélateur, que l'activation s'effectue sur la RdRP par l'intermédiaire d' une protéine de fusion, que l'activation sert à la détection de voies de transduction des signaux ou de la translocation de facteurs intra-cellulaires, que l'activation s'effectue par l'entrée d'une substance diffusante ou d'une toxine ou par une activation par une infection de la cellule hôte.

11. Procédé selon l'une ou plusieurs des revendications 1 à 10, qui est approprié
(a) pour l'amplification d'acides nucléiques, en particulier pour l'amplification d'ARN dans des cellules animales ; et/ou
(b) pour la découverte de mutations avantageuses dans la RdRP ou dans le gène étranger, sachant que les mutations avantageuses conduisent en particulier à une réplication améliorée du système, à un rendement amélioré du gène étranger ou à une toxicité moindre du gène étranger ; et/ou
(c) pour l'expression d'un gène étranger, y compris d'un gène révélateur, pour la détection de voies de transduction du signal ou de la translocation de facteurs intracellulaires; et/ou
(d) pour le contrôle de l'expression du gène.

12. Cellule animale à l'exception de cellules souches humaines embryonnaires, contenant
(i) une ARN-polymérase fonction de l'ARN (RdRP) d'un virus de plante et
(ii) un ARN qui contient des promoteurs ou des signaux cis-actifs, lesquels sont reconnus par la RdRP (ARN substrat),
comme décrit dans une ou plusieurs des revendications 1 à 11.

13. Transcrits isolés qui sont appropriés pour l'introduction
(i) d'une ARN-polymérase fonction de l'ARN (RdRP) d'un virus de plante et
(ii) d'un ARN qui contient des promoteurs ou des signaux cis-actifs, lesquels sont reconnus par la RdRP (ARN substrat),
comme décrit dans une ou plusieurs des revendications 1 à 11, dans des cellules animales à l'exception des cellules souches embryonnaires humaines et
(a) qui codent une RdRP ; et
(b) qui contiennent un gène étranger et au moins un promoteur qui est reconnu par la RdRP, sachant que les deux se présentent dans l'orientation anti-sens et que le gène étranger ne peut pas être exprimé sans la RdRP, mais est activé par son expression.

14. Transcrits isolés selon la revendication 13, dans lesquels la RdRP et les promoteurs proviennent d'un virus de plante, sachant que la polymérase provient de préférence d'un représentant de la famille des Tombusviridae, en particulier du virus Turnip-Crincle.

15. Transcrits isolés selon la revendication 13 ou 14,
(a) qui contiennent des promoteurs dont la force a été modifiée par mutagenèse en comparaison des promoteurs existant naturellement ; et/ou
(b) dont l'ARN amplifié porte des éléments IRES, un donneur et un accepteur de shunt ou un amplificateur de traduction pour l'amélioration de l'expression du gène étranger ; et/ou
(c) dont l'ARN amplifié contient de préférence un tractus polyadénylyle, est procédé par un ribozyme, contient des signaux pour l'emballage dans une enveloppe de virus et est emballé dans des enveloppes de virus, code pour un gène ou pour plusieurs gènes, code pour des gènes d'orientation différente, représente un ARN qui développe sans traduction un effet dans la cellule, comme des ribozymes, des ARN ribosomiques ou des constructions anti-sens et/ou code pour un ARN génomique ou sub-génomique d'un autre virus.

16. Utilisation des transcrits isolés selon l'une ou plusieurs des revendications 13 à 15 à une amplification in vitro d'acides nucléiques, en particulier pour l'amplification d'ARN dans des cellules animales à l'exception des cellules souches embryonnaires humaines.

17. Médicament pour la thérapie génétique, la vaccination ou la vaccination thérapeutique contenant un transcrit selon l'une ou plusieurs des revendications 13 à 15.
